# EUROPEAN PATENT APPLICATION

(11) **EP 4 400 509 A1**
(43) Date of publication of application: **17.07.2024**
(21) Application number: 22867403.2
(22) Date of filing: 08.09.2022
(51) Int. Cl.: C07K 7/08, C07K 14/00, C07K 16/00, C07K 16/28

(54) **COMPOUND, SALT OF COMPOUND, ANTIBODY MODIFICATION REAGENT, METHOD FOR PRODUCING MODIFIED ANTIBODY, AND MODIFIED ANTIBODY**

(30) Priority: 08.09.2021 JP 2021146504
(71) Applicant: Kagoshima University, Kagoshima-shi, Kagoshima 890-8580 (JP); Peptide Institute, Inc., Ibaraki-shi, Osaka 567-0085 (JP)
(72) Inventor: ITO, Yuji, Kagoshima-shi, Kagoshima 890-8580 (JP); YOSHIYA, Taku, Ibaraki-shi, Osaka 567-0085 (JP); TSUDA, Shugo, Ibaraki-shi, Osaka 567-0085 (JP)
(74) Representative: Marks & Clerk LLP
(86) International application number: PCT/JP2022/033713
(87) International publication number: WO 2023/038082

(57) **Abstract**

A compound represented by Formula I described below or a salt of the compound: 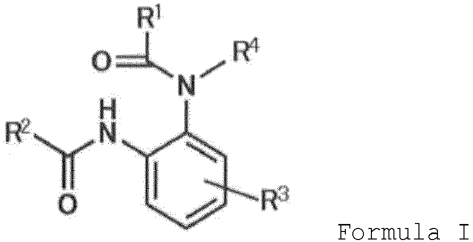 wherein R¹ is a substituent and R¹-H has a pKa of 4 to 14, R² or R³ has an IgG-binding peptide that specifically binds to an Fc region of an IgG, and R⁴ is H or a substituted or unsubstituted alkyl group having 1 to 8 carbon atoms.

## Description

### Technical Field

The present invention relates to a compound, a salt of the compound, an antibody modification reagent, a method for producing a modified antibody, and a modified antibody.

### Background

Antibody medicaments highly functionalized by attaching a drug, for example, antibody drug conjugates (ADCs) and the like have been developing. For modification of antibodies, various methods have been used such as a method for random modification of amino group and a method for thiol modification of a hinge site.

Patent Literature 1 to Patent Literature 3 disclose methods for chemical conjugation by affinity peptide (CCAP), which are a site-specific modification method for preparing a conjugate (complex) of an antibody and a drug or the like.

In the CCAP methods, an antibody modification reagent having an IgG-binding peptide (IgG-BP) that specifically binds to a Fc region of an IgG is reacted with an antibody. A predetermined amino acid residue of the IgG-BP is modified with N,N'-disuccinimidyl glutarate (disuccinimidyl glutarate (DSG)) or di(N-succinimidyl) 3,3'-dithiodipropionate (dithiobis(succinimidyl propionate) (DSP)), and thus the antibody modification reagent has N-hydroxysuccinimide (NHS) group. In the reaction between the antibody modification reagent and the antibody, the IgG-BP binds to a specific region of the Fc domain via NHS group.

In the CCAP method, after reacting the antibody modification reagent with the antibody, the IgG-BP remains in the modified antibody, and therefore in application of the modified antibody to medicaments and the like, the antigenicity of the remaining peptide after in-vivo administration has been an inherent concern. Meanwhile, Patent Literature 4 discloses another CCAP method in which the IgG-BP does not remain in the final product.

### Citation List

### Patent Literature

Patent Literature 1: International Publication No. WO 2016/186206
Patent Literature 2: International Publication No. WO 2017/217347
Patent Literature 3: International Publication No. WO 2018/230257
Patent Literature 4: International Publication No. WO 2018/199337

### Summary of Invention

In each antibody modification reagent used in the CCAP methods disclosed in Patent Literature 1 to Patent Literature 4, the NHS group tends to be hydrolyzed and is unstable, and the antibody modification reagent cannot withstand long-term storage. For industrial application of a CCAP method, improvement of the stability of an antibody modification reagent has been awaited.

The present invention has been made in view of the above circumstances, and an object of the present invention is to provide a compound that is chemically stable and can be stored for a long period of time, a salt of the compound, an antibody modification reagent, and a method for producing a modified antibody using the antibody modification reagent. Furthermore, an object of the present invention is to provide a novel modified antibody.

A compound according to a first aspect of the present invention is
represented by Formula I described below: 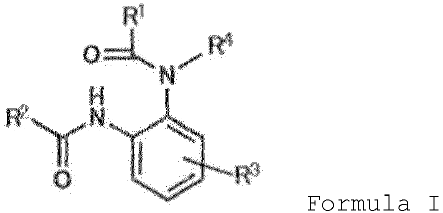 wherein R¹ is a substituent and R¹-H has a pKa of 4 to 14,
R² or R³ has an IgG-binding peptide that specifically binds to an Fc region of an IgG,
in a case where R² has the above-described IgG-binding peptide, R³ is absent or selected from the group consisting of substituted or unsubstituted alkyl groups having 1 to 8 carbon atoms, substituted or unsubstituted alkenyl groups having 2 to 8 carbon atoms, substituted or unsubstituted alkynyl groups having 2 to 8 carbon atoms, nitro group, halogens, and carboxamide groups,
in a case where R³ has the above-described IgG-binding peptide, R² is selected from the group consisting of substituted or unsubstituted alkyl groups having 1 to 8 carbon atoms, substituted or unsubstituted alkenyl groups having 2 to 8 carbon atoms, substituted or unsubstituted alkynyl groups having 2 to 8 carbon atoms, substituted or unsubstituted peptide chains having 2 to 50 amino acid residues, substituted or unsubstituted polymer chains having a polymerization degree of 2 to 50, and combinations thereof, and
R⁴ is H or a substituted or unsubstituted alkyl group having 1 to 8 carbon atoms.

A salt of the compound according to a second aspect of the present invention is a salt of the above-described compound according to the first aspect of the present invention.

R¹ may be nitrophenoxy group.

R² may have the above-described IgG-binding peptide, and R³ may be absent.

R² may be represented by Formula II described below: 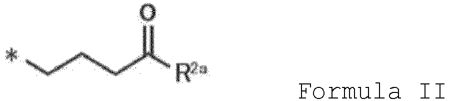 wherein R^{2a} represents the above-described IgG-binding peptide, and
* represents a carbon atom of carbonyl group in Formula I.

In a case where R² has the above-described IgG-binding peptide, the IgG-binding peptide may be modified with a drug, a reactive functional group, or a labeling substance.

R³ may have the above-described IgG-binding peptide, and R² may have a drug, a reactive functional group, or a labeling substance.

R³ may be represented by Formula III, IV, or V described below: 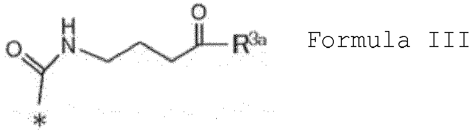 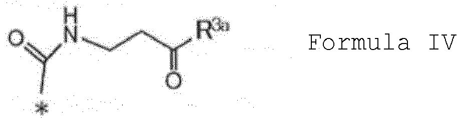 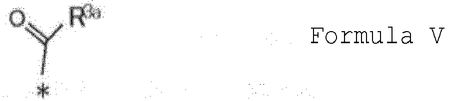 wherein R^{3a} represents the above-described IgG-binding peptide, and
* represents a carbon atom of benzene ring in Formula I.

The IgG-binding peptide described in the present description may have an amino acid sequence shown in any one of SEQ ID NOs: 1 to 115.

The above-described IgG-binding peptide may have an amino acid sequence shown in any one of SEQ ID NOs: 116 to 151.

An antibody modification reagent according to a third aspect of the present invention comprises the above-described compound according to the first aspect of the present invention or the above-described salt of the compound according to the second aspect of the present invention.

A method for producing a modified antibody according to a fourth aspect of the present invention comprises a reaction step of reacting the above-described antibody modification reagent according to the third aspect of the present invention with an IgG.

A modified antibody according to a fifth aspect of the present invention comprises a drug, a reactive functional group, or a labeling substance bound to Lys of an Fc region of an IgG in a monovalent or bivalent form via an atomic group containing Lys-Gly-Gly.

Amino group of a side chain of Lys contained in the above-described atomic group may be substituted with the drug, the reactive functional group, or the labeling substance described above.

The above-described reactive functional group may be azide group.

The above-described IgG may be a human IgG.

The above-described IgG may be tocilizumab or trastuzumab.

The compound, the salt of the compound, and the antibody modification reagent according to the present invention are chemically stable and can be stored for a long period of time. According to the present invention, a method for producing a modified antibody using the antibody modification reagent is provided. According to the present invention, a novel modified antibody is provided.

### Brief Description of Drawings

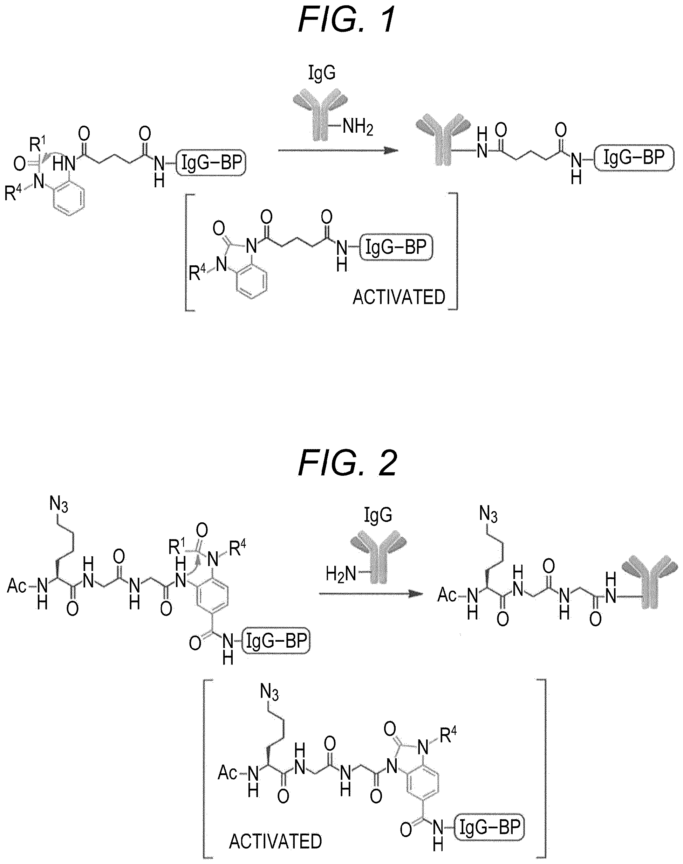

Fig. 1 is a view schematically showing a reaction of modifying an antibody with an IgG-BP using a compound according to an embodiment of the present invention.
Fig. 2 is a view schematically showing a reaction of modifying an antibody using a compound according to an embodiment of the present invention.

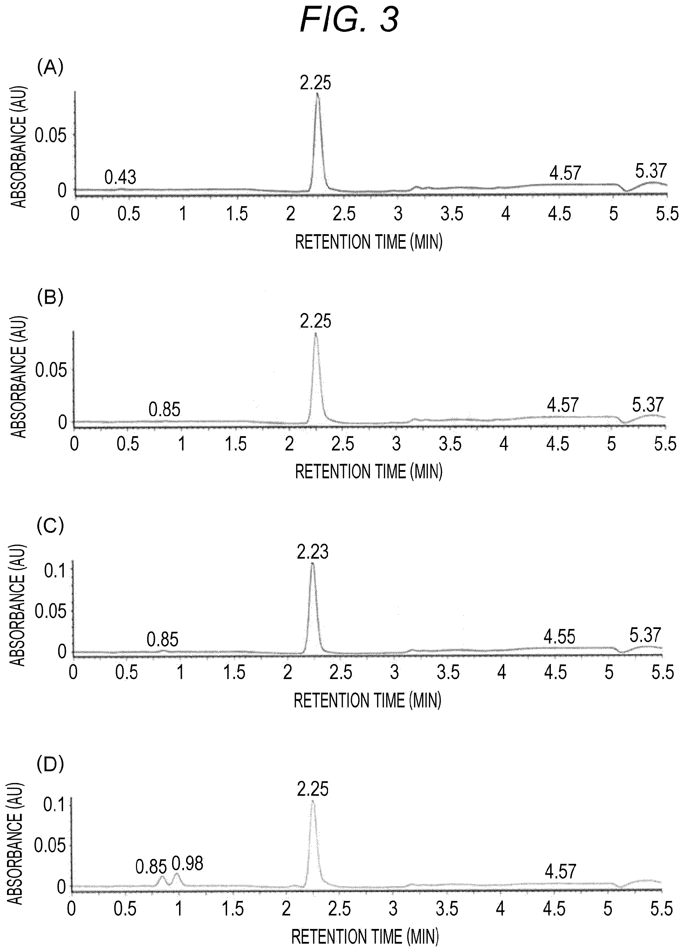

Fig. 3 shows graphs of results of analyzing reaction solutions of a compound according to Example 1 and an IgG by a liquid chromatography mass spectrometer (LC-MS) in Example 3. (A), (B), (C), and (D) are graphs showing elution chromatograms of the reaction solutions at pH 5.5, 7.0, 8.0, and 8.9, respectively.

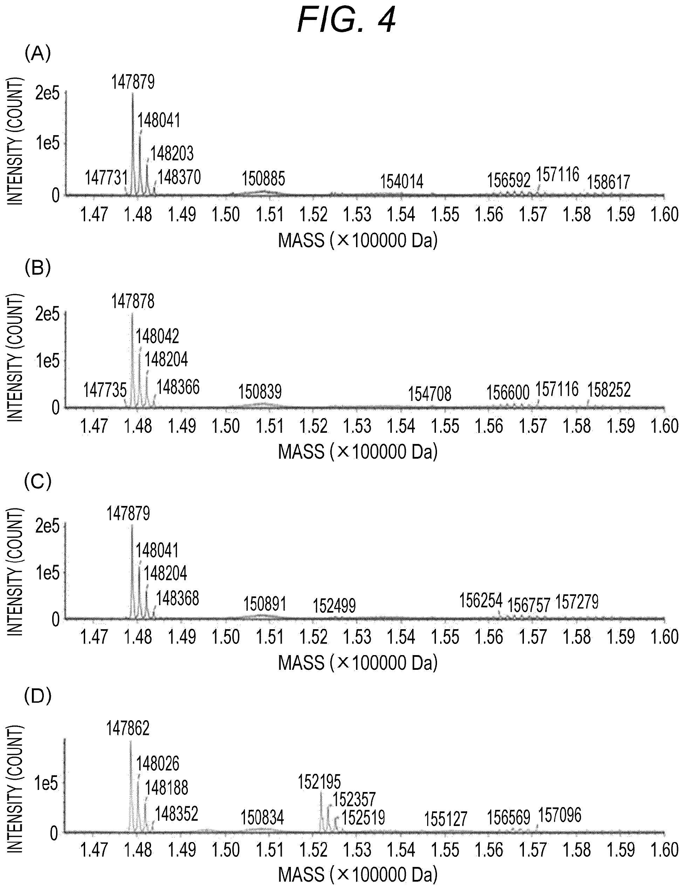

Fig. 4 shows graphs of results of analyzing the reaction solutions of the compound according to Example 1 and the IgG by an LC-MS in Example 3. (A), (B), (C), and (D) are graphs showing results of mass spectrometry of peaks of the IgG in the reaction solutions at pH 5.5, 7.0, 8.0, and 8.9, respectively.

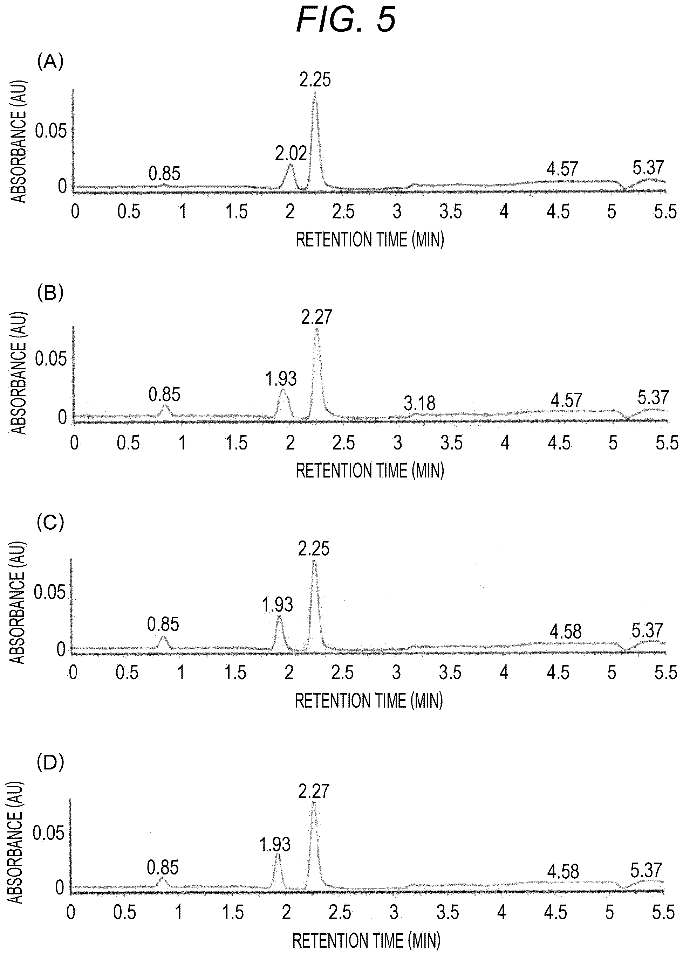

Fig. 5 shows graphs of results of analyzing reaction solutions of a compound according to Example 2 and an IgG by an LC-MS in Example 3. (A), (B), (C), and (D) are graphs showing elution chromatograms of the reaction solutions at pH 5.5, 7.0, 8.0, and 8.9, respectively.

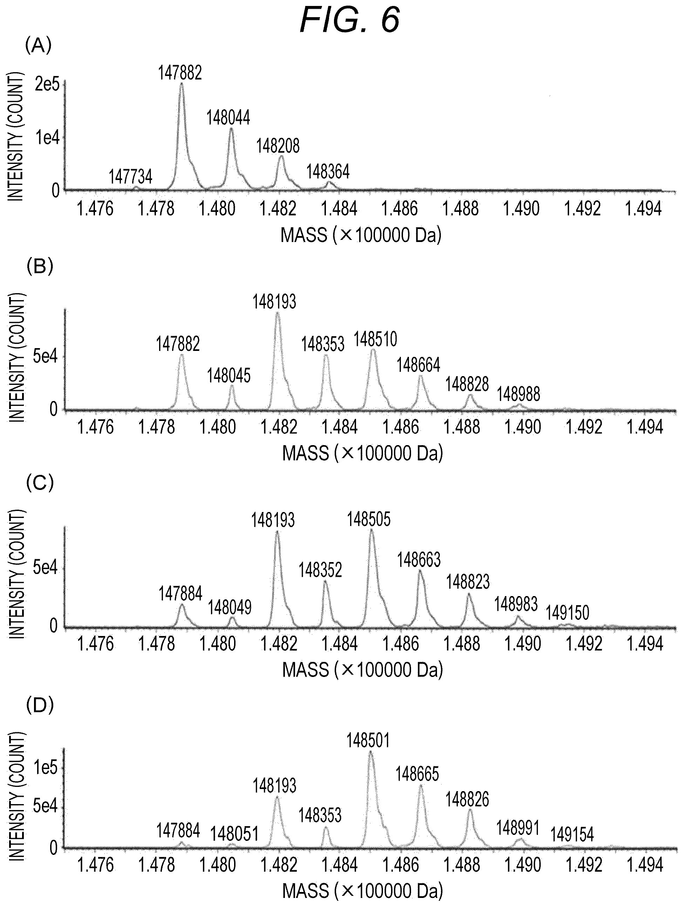

Fig. 6 shows graphs of results of analyzing the reaction solutions of the compound according to Example 2 and the IgG by an LC-MS in Example 3. (A), (B), (C), and (D) are graphs showing results of mass spectrometry of peaks of the IgG in the reaction solutions at pH 5.5, 7.0, 8.0, and 8.9, respectively.

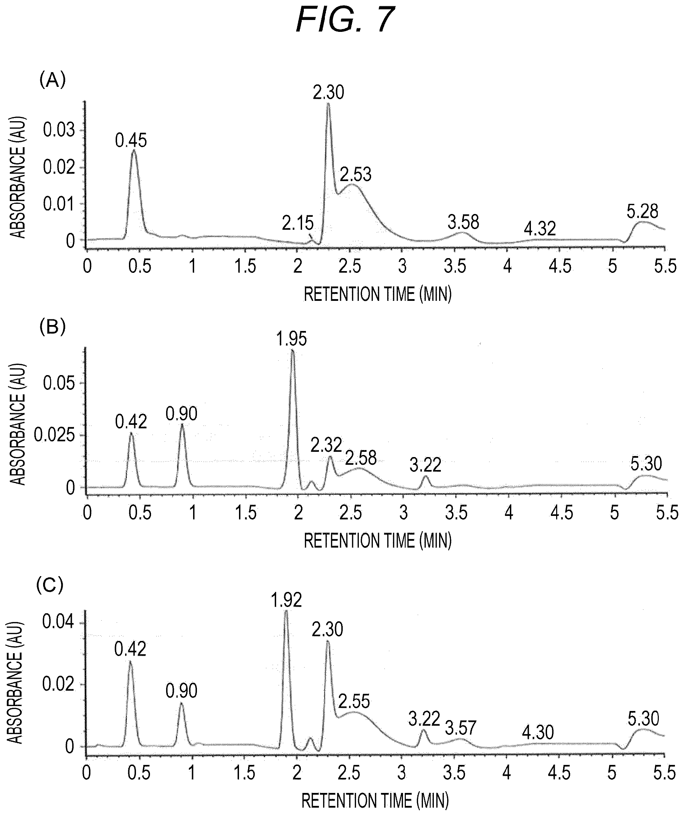

Fig. 7 shows graphs of results of analyzing reaction solutions of the compound according to Example 2 and mouse IgG by an LC-MS in Example 4. (A), (B), and (C) are graphs showing elution chromatograms of an antibody solution having a pH of 8.0 before addition of the compound, a solution having a pH of 8.0 2 hours after reaction, and a solution having a pH of 8.9 2 hours after reaction, respectively.

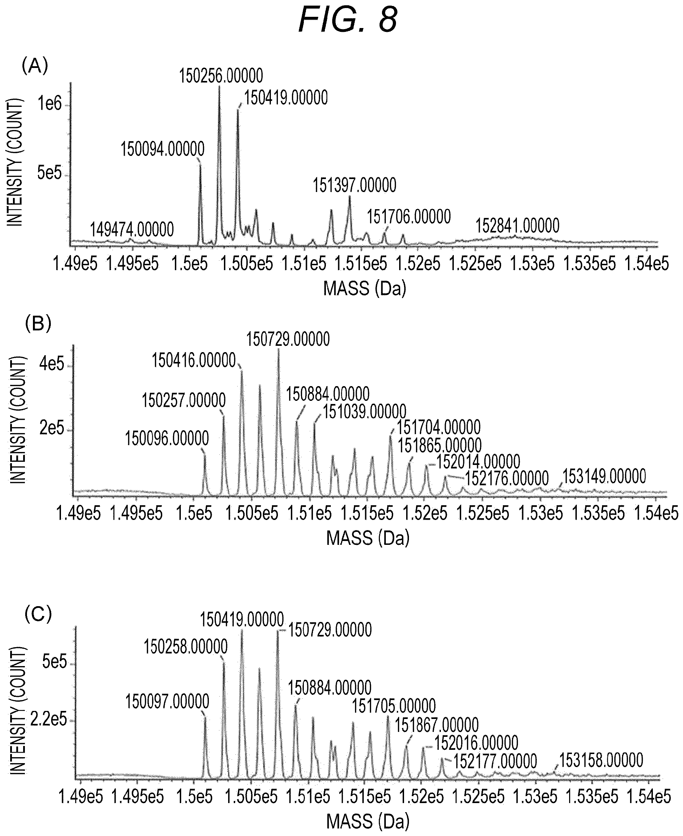

Fig. 8 shows graphs of results of analyzing the reaction solutions of the compound according to Example 2 and mouse IgG by an LC-MS in Example 4. (A), (B), and (C) are graphs showing results of mass spectrometry of peaks of the IgG in the antibody solution having a pH of 8.0 before addition of the compound, the solution having a pH of 8.0 2 hours after reaction, and the solution having a pH of 8.9 2 hours after reaction, respectively.

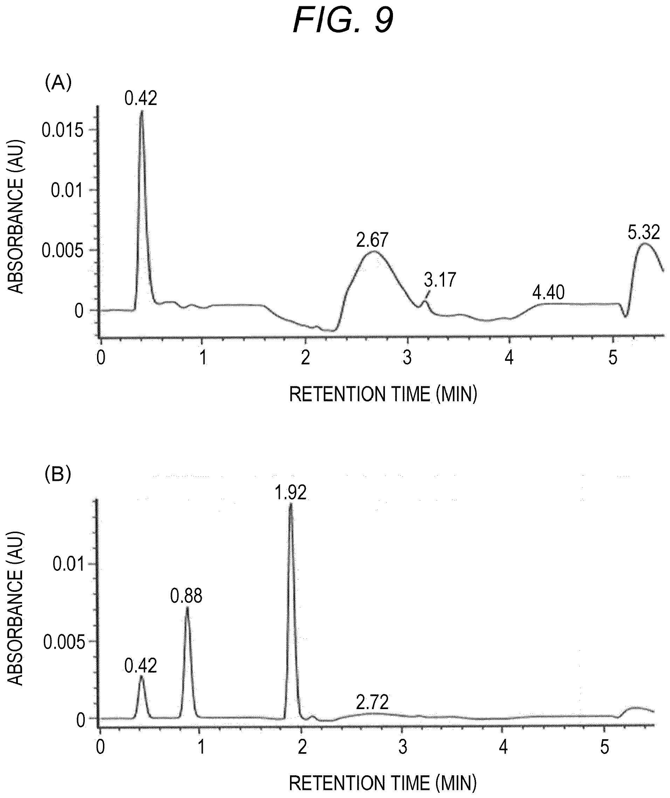

Fig. 9 shows graphs of results of analyzing reaction solutions of the compound according to Example 2 and rabbit IgG by an LC-MS in Example 5. (A) and (B) are graphs showing elution chromatograms of an antibody solution having a pH of 8.0 before addition of the compound and a solution having a pH of 8.0 24 hours after reaction, respectively.

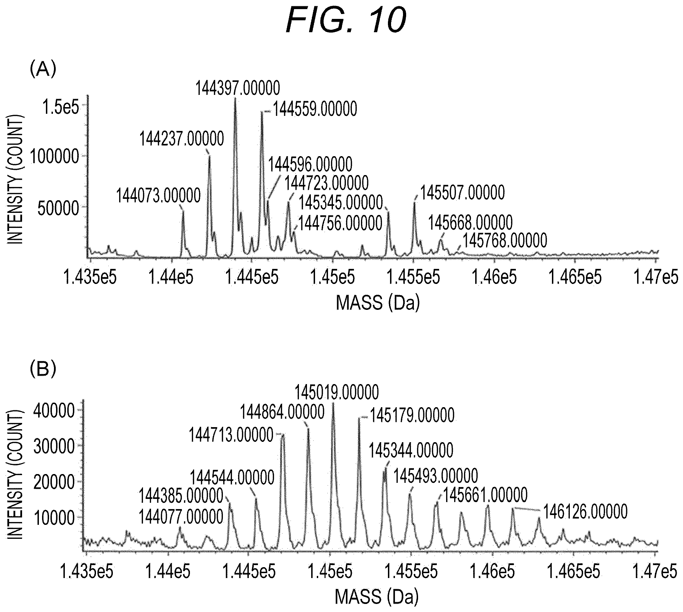

Fig. 10 shows graphs of results of analyzing the reaction solutions of the compound according to Example 2 and rabbit IgG by an LC-MS in Example 5. (A) and (B) are graphs showing results of mass spectrometry of peaks of the IgG in the antibody solution having a pH of 8.0 before addition of the compound and the solution having a pH of 8.0 24 hours after reaction, respectively.

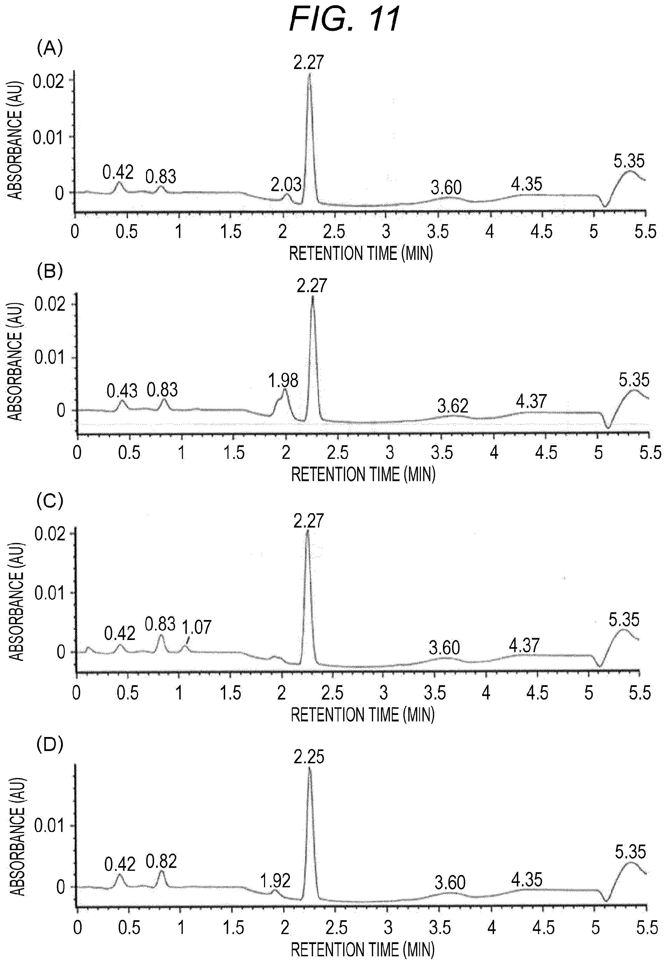

Fig. 11 shows graphs of results of analyzing reaction solutions of a compound according to Example 6 and a human IgG by an LC-MS in Example 8. (A), (B), (C), and (D) are graphs showing elution chromatograms of the reaction solutions at pH 5.5, 7.0, 8.0, and 8.9, respectively.

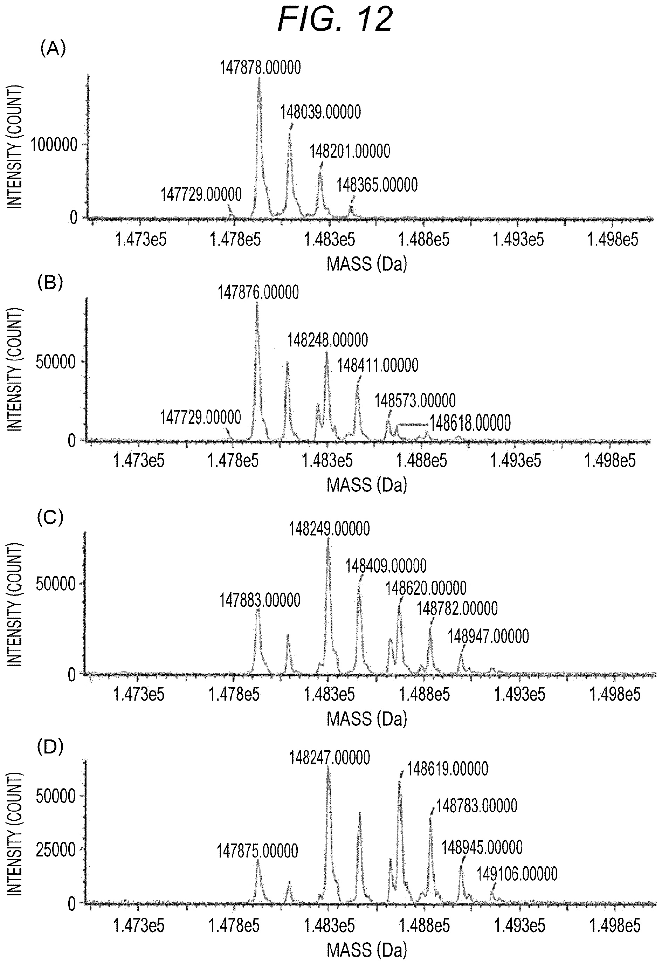

Fig. 12 shows graphs of results of analyzing the reaction solutions of the compound according to Example 6 and the human IgG by an LC-MS in Example 8. (A), (B), (C), and (D) are graphs showing results of mass spectrometry of peaks of the IgG in the reaction solutions at pH 5.5, 7.0, 8.0, and 8.9, respectively.

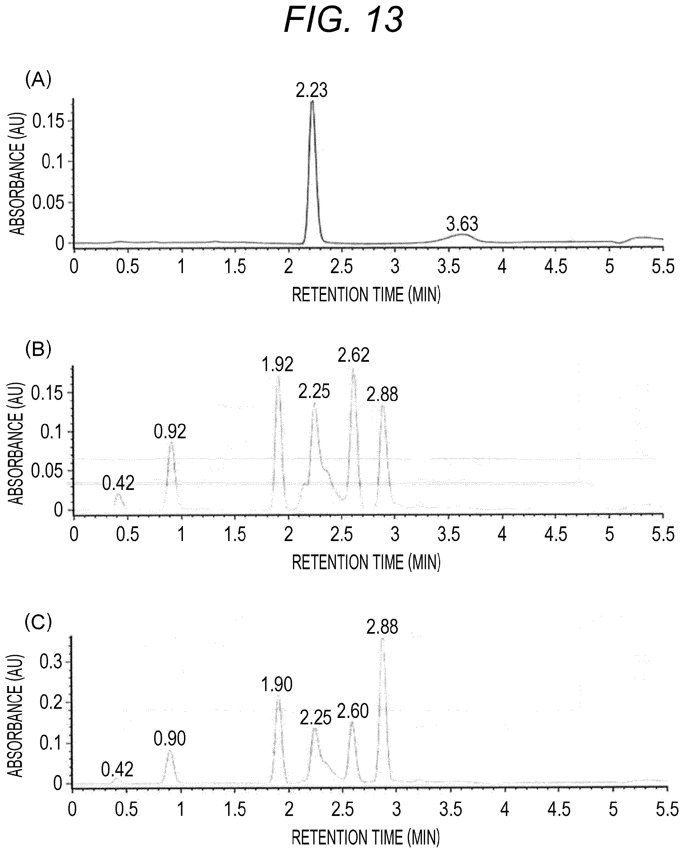

Fig. 13 shows graphs of results of LC-MS of reaction solutions of a compound according to Example 7 and humanized IgG in Example 9. (A), (B), and (C) are graphs showing elution chromatograms of an antibody solution before addition of the compound, a solution having a pH of 8.0 24 hours after reaction, and a solution having a pH of 8.9 24 hours after reaction, respectively.

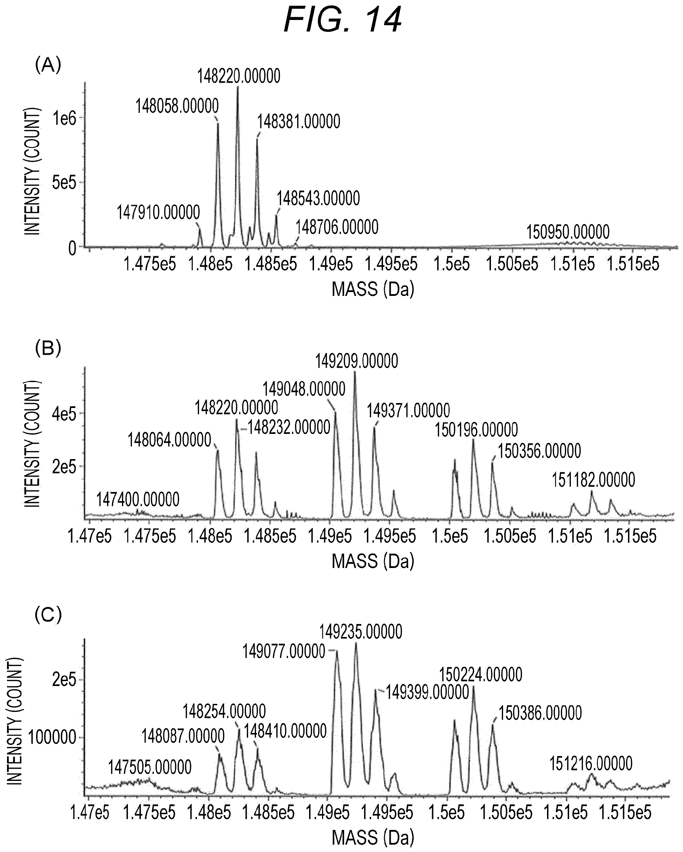

Fig. 14 shows graphs of results of LC-MS of the reaction solutions of the compound according to Example 7 and the humanized IgG in Example 9. (A), (B), and (C) are graphs showing results of mass spectrometry of peaks of the IgG in the antibody solution before addition of the compound, the solution having a pH of 8.0 24 hours after reaction, and the solution having a pH of 8.9 24 hours after reaction, respectively.

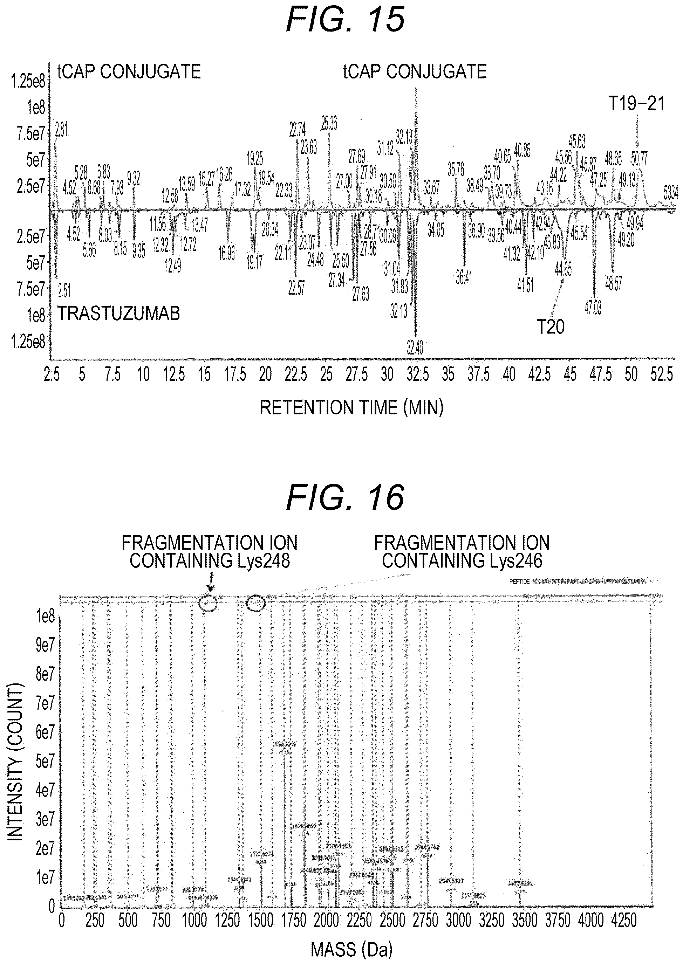

Fig. 15 is a graph showing a result of peptide mapping of trastuzumab reacted with the compound according to Example 2 in Test Example 2.
Fig. 16 is a graph showing a result of tandem mass spectrometry (MS/MS) for a peak eluted in trastuzumab reacted with the compound according to Example 2 in Test Example 2.

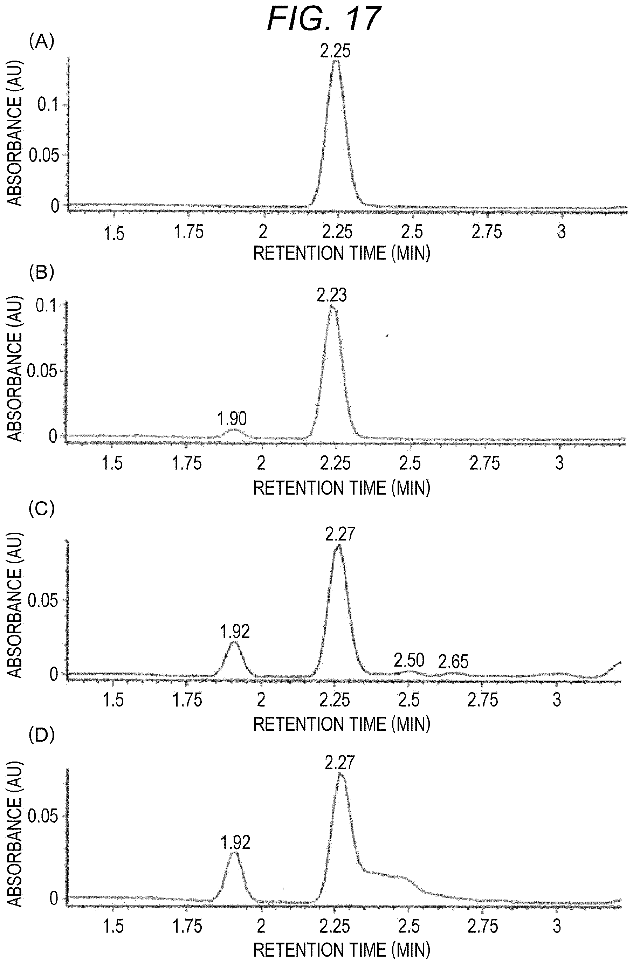

Fig. 17 shows graphs of results of LC-MS in Example 10. (A), (B), (C), and (D) are graphs showing elution chromatograms of trastuzumab (unmodified antibody), azidated trastuzumab modified with the compound according to Example 2 (azidated KGG-modified antibody), a reaction product of an anticancer drug and azidated trastuzumab, and a reaction product of azidated trastuzumab and a fluorescent agent, respectively.

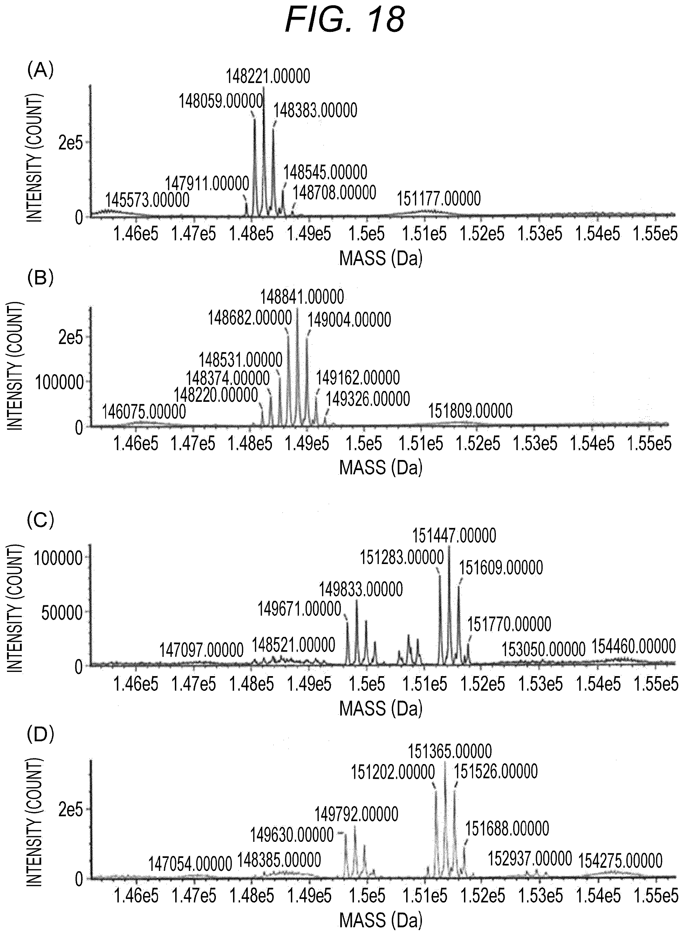

Fig. 18 shows graphs of results of LC-MS in Example 10. (A), (B), (C), and (D) are graphs showing results of mass spectrometry of peaks of antibodies eluted in the unmodified antibody, the azidated KGG-modified antibody, the reaction product of an anticancer drug and azidated trastuzumab, and the reaction product of azidated trastuzumab and a fluorescent agent, respectively.

### Description of Embodiments

Embodiments according to the present invention will be described with reference to the drawings. Note that the present invention is not limited by the following embodiments and drawings. In the following embodiments, expressions "having", "including", "containing", and the like also includes the meaning of "consisting of" and "formed of".

### (Compound and Its Salt)

A compound according to the present embodiment is represented by Formula I described below.

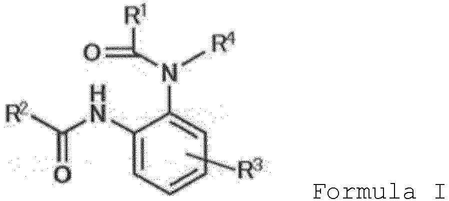

In Formula I, R¹ is a substituent, and R¹-H has an acid dissociation constant (pKa) of 4 to 14, 4 to 12, or 4 to 10, preferably 5 to 9. Here, the pKa is a pKa at room temperature (for example, 25°C). The above-described compound becomes an activated intermediate by elimination of R¹. The pKa of R¹-H determines the ease of the elimination, that is, the ease of transition to the activated intermediate. If the pKa is high, the transition is less likely to occur, and if the pKa is low, the transition is likely to occur, but if the pKa is too low, the compound is unstable and may be hydrolyzed. R¹-H is preferably a phenol. Examples of R¹-H include salicylic acid, phenol, 4-fluorophenol, 4-nitrophenol, 2,6-dichlorophenol, N-hydroxysuccinimide, 2,3,5,6-tetrafluorophenol, pentafluorophenol, aminophenol, and dinitrophenol. Suitably, R¹-H is 4-nitrophenol and R¹ is nitrophenoxy group.

R² or R³ has an IgG-BP that specifically binds to an Fc region of an IgG. The "IgG" may be an IgG of a mammal, for example, a primate such as a human or a chimpanzee, an experimental animal such as a rat, a mice, or a rabbit, a domestic animal such as a pig, a cow, a horse, a sheep, or a goat, or a pet animal such as a dog or a cat, and is preferably a human IgG (IgG1, IgG2, IgG3, or IgG4). The IgG is preferably human IgG1, IgG2, or IgG4, or rabbit IgG, and particularly preferably human IgG1, IgG2, or IgG4. The "Fc region of an IgG" typically means a C-terminal fragment obtained as a treated product of an IgG with protease papain.

In a case where R² has the IgG-BP, R³ is absent or selected from the group consisting of substituted or unsubstituted alkyl groups having 1 to 8 carbon atoms, substituted or unsubstituted alkenyl groups having 2 to 8 carbon atoms, substituted or unsubstituted alkynyl groups having 2 to 8 carbon atoms, nitro group, halogens, and carboxamide groups. In a case where R² has IgG-BP, R³ is preferably absent.

The term "alkyl groups" means saturated hydrocarbon groups. The alkyl groups include cyclic alkyl groups (including fused bicyclic alkyl groups), linear or branched alkyl groups, and linear or branched alkyl groups substituted with a cyclic alkyl group, satisfying the condition of the number of carbon atoms. Examples of the alkyl groups include methyl group, ethyl group, n-propyl group, iso-propyl group, cyclopropyl group, n-butyl group, iso-butyl group, sec-butyl group, t-butyl group, n-pentyl group, 1,1-dimethylpropyl group, 1,2-dimethylpropyl group, 2,2-dimethylpropyl group, 1-ethylpropyl group, 2-ethylpropyl group, n-hexyl group, cyclohexyl group, cyclooctyl group, and 1-methyl-2-ethylpropyl group.

The term "alkenyl" means a hydrocarbon group having at least one double bond. The alkenyl groups include both linear alkenyl groups and branched alkenyl groups. Examples of the alkenyl include ethenyl group, n-propenyl group, iso-propenyl group, n-butenyl group, iso-butenyl group, sec-butenyl group, t-butenyl group, n-pentenyl group, 1,1-dimethylpropenyl group, 1,2-dimethylpropenyl group, 2,2-dimethylpropenyl group, 1-ethylpropenyl group, 2-ethylpropenyl group, n-hexenyl group, and 1-methyl-2-ethylpropenyl group.

The term "alkynyl" means a hydrocarbon group having at least one triple bond. The alkynyl groups include both linear alkynyl groups and branched alkynyl groups. Examples of the alkynyl include ethynyl group, n-propynyl group, iso-propynyl group, n-butynyl group, iso-butynyl group, sec-butynyl group, t-butynyl group, and n-pentynyl group.

In a case where R² has the IgG-BP, R² may be the IgG-BP. Alternatively, R² may have a linker (Linker), and may be *-(Linker)-(IgG-BP) wherein * represents a carbon atom of carbonyl group in Formula I. The linker is not particularly limited as long as the compound maintains the specific binding ability to the Fc region, and is, for example, selected from the group consisting of -NH-, -O-, - S-, -C(=O)-NH-, -NH-C(=O)-, -O-, -C(=O)-O-, -O-C(=O)-, -S-, -C(=O)-, polyoxyalkylene groups, amino acid residues, peptide chains, polyethylene glycol (PEG) chains, and combinations thereof.

Suitably, R² is represented by Formula II wherein R^{2a} represents the IgG-BP.

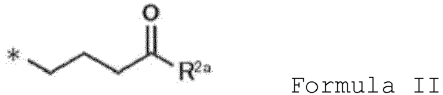

The IgG-BP in R² is bound to the carbon atom of the carbonyl group in Formula I or the linker via the main chain or a side chain of any amino acid residue. The IgG-BP in R² is preferably bound to the carbon atom of the carbonyl group in Formula I or the linker via the N-terminal main chain or side chain amino group.

The IgG-BP in R² may be modified with a drug, a reactive functional group, or a labeling substance. Examples of the drug include anticancer drugs such as auristatins such as auristatin E, maytansine, emtansine, doxorubicin, bleomycin, and their derivatives, drugs that binds to a receptor of the blood-brain barrier to promote migration into the central nerve, and targeting agents such as drugs that binds to a cancer cell or the like to enable migration of the IgG into the cell.

Examples of the reactive functional group include imidazole group, hydroxy group, amino group, thiol group, halogen atoms, sulfonic acid ester groups, epoxy group, isocyanate group, azide group, vinyl group, maleimide group, sulfhydryl group, ethynyl group (-C≡CH), dienes, alkynes, bicyclo(6.1.0)nonyne (BCN), dibenzocyclooctyne (DBCO) group, trans-cyclooctene (TCO), and tetrazine. Preferably, the reactive functional group is azide group, and the IgG-BP can be modified with a desired molecule by a click reaction with an alkyne, DBCO group, or the like.

The labeling substance is not limited, and is, for example, a fluorescent dye, a chemiluminescent dye, a radioisotope, a luminescent protein, biotin, a fluorescent protein such as green fluorescent protein, or an enzyme such as peroxidase. The labeling substance is preferably fluorescein (FAM) or a fluorescein derivative such as FITC, rhodamine or a rhodamine derivative such as tetramethylrhodamine, or a fluorescent dye such as Texas Red.

Fig. 1 shows a reaction of modifying an IgG with the compound according to the present embodiment in which R² has the IgG-BP. The compound has an active ester precursor represented by Formula I, and therefore can add the IgG-BP to a predetermined amino acid residue of the IgG, particularly Lys residue, via an activated compound under a neutral condition, preferably a weak alkaline condition.

Next, a case where R³ has the IgG-BP will be described. In a case where R³ has the IgG-BP, R² is selected from the group consisting of substituted or unsubstituted alkyl groups having 1 to 8 carbon atoms, substituted or unsubstituted alkenyl groups having 2 to 8 carbon atoms, substituted or unsubstituted alkynyl groups having 2 to 8 carbon atoms, substituted or unsubstituted peptide chains having 2 to 50 amino acid residues, substituted or unsubstituted polymer chains having a polymerization degree of 2 to 50, and combinations thereof. The number of amino acid residues in each peptide chain is preferably 2 to 30, 2 to 20, 2 to 10, or 2 to 5, and more preferably 3. Specific examples of the peptide chains include Lys-Gly-Gly. The polymer chains are not particularly limited as long as they are a known polymer chain, and examples thereof include PEG. The substituted peptide chains include a peptide chain in which a substituent is bound to the main chain or a side chain. The substituted polymer chains include a polymer chain in which a substituent is bound to the main chain or a side chain.

In a case where R³ has the IgG-BP, R³ may be the IgG-BP. R³ may have a linker as in R² described above.

The IgG-BP in R³ is bound to a carbon atom of benzene ring in Formula I or the linker via the main chain or a side chain of any amino acid residue. The IgG-BP in R³ is preferably bound to the carbon atom of the carbonyl group in Formula I or the linker via the N-terminal main chain or side chain amino group.

Suitably, R³ is represented by Formula III, IV, or V wherein R^{3a} represents the IgG-BP. In Formulas III, IV, and V, * represents the carbon atom of the benzene ring in Formula I.

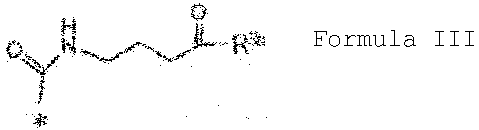

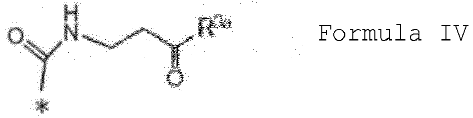

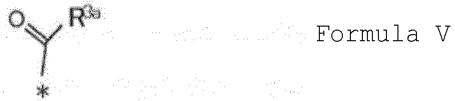

In a case where R³ has the IgG-binding peptide, R² may have the drug, the reactive functional group, or the labeling substance described above. The drug, the reactive functional group, or the labeling substance in R² is bound to, for example, an alkyl group, an alkenyl group, an alkynyl group, a peptide chain, or a polymer chain. In a case where R² has, for example, Lys-Gly-Gly as a peptide chain, the amino group of the side chain of Lys of the peptide chain may be substituted with the drug, the reactive functional group, or the labeling substance.

Fig. 2 shows a reaction of modifying an IgG with the compound according to the present embodiment in which R³ has the IgG-BP. The compound has an active ester precursor represented by Formula I, and therefore binds to the IgG via an activated compound. In the compound in which R³ has the IgG-BP, unlike in a case where R² has the IgG-BP, a substituent containing R² is added to a predetermined amino acid residue of the IgG, and R³ containing the IgG-BP is not added to the IgG. Therefore, in a case where R³ has the IgG-BP, R² preferably has the drug, the reactive functional group, or the labeling substance described above. In this way, the IgG can be easily modified with the drug, the reactive functional group, or the labeling substance.

In the CCAP method in which an IgG-BP does not remain in an IgG disclosed in Patent Literature 4 described above and the like, an elimination reaction of the IgG-BP is required after modification of the IgG. Meanwhile, if the compound according to the present embodiment is used, an elimination reaction of the IgG-BP is completed simultaneously with modification of the IgG.

The IgG-BP is preferably a peptide that binds to a site selected from Lys248, Lys246, Lys338, Lys288, Lys290, Lys360, Lys414, and Lys439 according to Eu numbering in Fc or an adjacent region of the site, preferably Lys248 or an adjacent region of Lys248, or binds to a binding region of Protein A. For example, the IgG-BP may be a partial peptide of Protein A having Fc-binding ability or a variant of the partial peptide. Specific examples of such peptides are described in International Publication No. WO 2008/054030, International Publication No. WO 2013/027796, Patent Literature 1 to 3 above, and the like. The IgG-BP can be appropriately prepared in accordance with a known peptide synthesis method, for example, a solid-phase peptide synthesis method or a method described in each literature.

More specifically, R² and R³ are exemplified in the following (i) to (iii).
(i) An IgG-BP-containing substituent represented by Formula (PI) described below:

   NH₂-(Linker)-(X¹₁₋₃)-C-(X²)-(X³)-(X⁴)-(X⁵)-G-(X⁶)-L-(X⁷)-W-C-(X⁸₁₋₃)-(Linker) ••• Formula (PI)

   wherein each (Linker) is independently the same as or different from another linker or is independently absent, the linker is RRRGS, EEGGS, (GSGGS)₁₋₃, or (PEG)₁₋₁₀, preferably (PEG)₁₋₈ or (PEG)₂₋₁₀, and more preferably (PEG)₄,
   1 to 3 X¹s, X², X³, X⁴, X⁵, X⁶, X⁷, and 1 to 3 X⁸s each independently represent the same or different amino acid residues,
   X¹s, X², X³, and X⁸s each independently represent the same or different any amino acid residues other than C,
   X⁴ is H, R, S, or D,
   X⁵ is one amino acid residue selected from K, C, D, E, R, V, F, L, 2-aminosuberic acid, Dpr, Orn, AcOrn, AcDab, Dab, Nle, Nva, Tle, Ala(t-Bu), and Cha,
   X⁶ is E, N, R, or D, and
   X⁷ is I or V.
   Dab, Tle, and Cha mean 2,4-diaminobutanoic acid, tert-leucine, and β-cyclohexyl-L-alanine, respectively. Ac and t-Bu mean having acetyl group, and tert-butyl group, respectively.

In Formula (PI), for binding, amino group may be bound to the terminal (-COOH) of the C-terminal amino acid residue in Formula (PI) to form (-C(=O)NH₂) group. In Formula (PI), the N-terminal amino group may be acetylated. In this case, Lys residue may be introduced at an appropriate position near the N-terminal in Linker.

Preferred examples of the IgG-BP contained in the substituent represented by Formula (PI) include the following peptides.
1. X¹₁₋₃ is an amino acid sequence represented by (S, G, F, or none)-(D, G, A, S, P, Hcy, or none)-(S, D, T, N, E, or R).
2. X¹₁₋₃ is D, GPD, R, GPR, SPD, GDD, GPS, SDD, RGN, G-Hcy-D, RGP, or GPD.
3. X¹₁₋₃ is D or GPD.
4. X² is A, S, or T.
5. X² is A or T.
6. X² is A.
7. X³ is Y or W.
8. X³ is Y.
9. X⁴ is H.
10. X⁵ is one amino acid residue selected from A, R, K, C, D, E, L, 2-aminosuberic acid, Dpr, R, F, 2-aminosuberic acid, , Dpr, AcOrn, AcDab, Dab, Nle, Nva, Ala(t-Bu), and Cha.
11. X⁵ is K, R, or AcOrn.
12. X⁵ is one amino acid residue selected from V, Dab, F, R, L, Nva, Nle, Ala(t-Bu), and Cha.
13. X⁵ is one amino acid residue selected from F, R, L, Nva, Nle, Ala(t-Bu), and Cha.
14. X⁵ is one amino acid residue selected from L, Ala(t-Bu), and Cha.
15. X⁶ is E or N.
16. X⁶ is E.
17. X⁷ is V.
18. X⁸₁₋₃ is (S, T, or D) - (H, G, Y, T, N, D, F, Hcy, or none) - (Y, F, H, M, or none) .
19. X⁸₁₋₃ is T, TFH, S, SFH, THH, TFY, TYH, or T-Hcy-H.
20. X⁸₁₋₃ is T or TFH.

The IgG-BP contained in the substituent represented by Formula (PI) may be any one or a combination of two or more described in the above conditions, and for example, may be a peptide satisfying the following conditions: 8 and 9, 8 and 17, 9 and 17, 8, 9, and 17, or a combination of one of these with any one of 10 to 14.

More specific examples of the IgG-BP can include the following peptides (X⁵ is the same as described above, and may have an NH₂-(Linker)-group at the N-terminus, and may have -NH₂ group or a -(Linker)-NH₂ group at the C-terminus):
1) DCAYHX⁵GELVWCT (SEQ ID NO: 1)
2) GPDCAYHX⁵GELVWCTFH (SEQ ID NO: 2)
3) RCAYHX⁵GELVWCS (SEQ ID NO: 3)
4) GPRCAYHX⁵GELVWCSFH (SEQ ID NO: 4)
5) SPDCAYHX⁵GELVWCTFH (SEQ ID NO: 5)
6) GDDCAYHX⁵GELVWCTFH (SEQ ID NO: 6)
7) GPSCAYHX⁵GELVWCTFH (SEQ ID NO: 7)
8) GPDCAYHX⁵GELVWCSFH (SEQ ID NO: 8)
9) GPDCAYHX⁵GELVWCTHH (SEQ ID NO: 9)
10) GPDCAYHX⁵GELVWCTFY (SEQ ID NO: 10)
11) SPDCAYHX⁵GELVWCTFY (SEQ ID NO: 11)
12) SDDCAYHX⁵GELVWCTFY (SEQ ID NO: 12)
13) RGNCAYHX⁵GQLVWCTYH (SEQ ID NO: 13)
14) G-Hcy-DCAYHX⁵GELVWCT-Hcy-H (SEQ ID NO: 14)
15) RRGPDCAYHX⁵GELVWCTFH (SEQ ID NO: 15)
16) DCTYHX⁵GNLVWCT (SEQ ID NO: 16)
17) DCAYHX⁵GNLVWCT (SEQ ID NO: 17)
18) DCTYHX⁵GELVWCT (SEQ ID NO: 18)
19) DCAWHX⁵GELVWCT (SEQ ID NO: 19)
20) DCTYTX⁵GNLVWCT (SEQ ID NO: 20)
21) DCAYTX⁵GNLVWCT (SEQ ID NO: 21)
22) DCSYTX⁵GNLVWCT (SEQ ID NO: 22)
23) DCTWTX⁵GNLVWCT (SEQ ID NO: 23)
24) DCTYHX⁵GNLVWCT (SEQ ID NO: 24)
25) DCTYRX5GNLVWCT (SEQ ID NO: 25)
26) DCTYSX⁵GNLVWCT (SEQ ID NO: 26)
27) DCTYTX⁵GNLVWCT (SEQ ID NO: 27)
28) DCTYTX⁵GELVWCT (SEQ ID NO: 28)
29) DCTYTX⁵GRLVWCT (SEQ ID NO: 29)
30) DCTYTX⁵GDLVWCT (SEQ ID NO: 30)
31) DCTYTX⁵GNLIWCT (SEQ ID NO: 31)
32) DCAYHRGELVWCT (SEQ ID NO: 32)
33) GPDCAYHRGELVWCTFH (SEQ ID NO: 33)
34) RCAYHRGELVWCS (SEQ ID NO: 34)
35) GPRCAYHRGELVWCSFH (SEQ ID NO: 35)
36) SPDCAYHRGELVWCTFH (SEQ ID NO: 36)
37) GDDCAYHRGELVWCTFH (SEQ ID NO: 37)
38) GPSCAYHRGELVWCTFH (SEQ ID NO: 38)
39) GPDCAYHRGELVWCSFH (SEQ ID NO: 39)
40) GPDCAYHRGELVWCTHH (SEQ ID NO: 40)
41) GPDCAYHRGELVWCTFY (SEQ ID NO: 41)
42) SPDCAYHRGELVWCTFY (SEQ ID NO: 42)
43) SDDCAYHRGELVWCTFY (SEQ ID NO: 43)
44) DCTYHRGNLVWCT (SEQ ID NO: 44)
45) DCAYHRGNLVWCT (SEQ ID NO: 45)
46) DCTYHRGELVWCT (SEQ ID NO: 46)
47) DCAWHRGELVWCT (SEQ ID NO: 47)
48) DCTYTNGNLVWCT (SEQ ID NO: 48)
49) DCAYTNGNLVWCT (SEQ ID NO: 49)
50) DCSYTNGNLVWCT (SEQ ID NO: 50)
51) DCTWTNGNLVWCT (SEQ ID NO: 51)
52) DCTYHNGNLVWCT (SEQ ID NO: 52)
53) DCTYRNGNLVWCT (SEQ ID NO: 53)
54) DCTYSNGNLVWCT (SEQ ID NO: 54)
55) DCTYTRGNLVWCT (SEQ ID NO: 55)
56) DCTYTNGELVWCT (SEQ ID NO: 56)
57) DCTYTNGRLVWCT (SEQ ID NO: 57)
58) DCTYTNGDLVWCT (SEQ ID NO: 58)
59) DCTYTNGNLIWCT (SEQ ID NO: 59)

Examples of R² and R³ include substituents having the following structures.
60) GSGGS-GPDCAYHRGELVWCTFH-NH₂ (SEQ ID NO: 60)
(PEG)₄-GPDCAYHRGELVWCTFH-NH₂ (SEQ ID NO: 33)
61) GSGGS-DCAYHRGELVWCT-NH₂ (SEQ ID NO: 61)
(PEG)₄-DCAYHRGELVWCT-NH₂ (SEQ ID NO: 32)

In the present description, examples of the peptide and the IgG-BP include a peptide to which functional group Z is bound. For example, in the substituent represented by Formula (PI), functional group Z may be bound to the end of (Linker). Examples of such a substituent can include the following substituents.
62) Acetyl-K(Z)-RRRGS-GPDCAYHKGELVWCTFH-NH₂ (SEQ ID NO: 62)
63) Acetyl-K(Z)-EEGGS-GPDCAYHKGELVWCTFH-NH₂ (SEQ ID NO: 63)
64) Acetyl-K(Z)-(PEG)₄-GPDCAYHKGELVWCTFH-NH₂ (SEQ ID NO: 64)

R² and R³ may be a substituent in which maleimide group, DBCO group, tetrazine group, or TCO group is bound to the N-terminus or PEG of a peptide described below, or may be a substituent in which NH₂ group is bound to the C-terminus.
65) RRRGS-GPDCAYHKGELVWCTFH (SEQ ID NO: 65)
66) EEGGS-GPDCAYHKGELVWCTFH (SEQ ID NO: 66)
(PEG)₄-GPDCAYHKGELVWCTFH (SEQ ID NO: 64)

Examples of the substituent in which functional group Z is bound to the end of (Linker) can include the following peptides.
67) Acetyl-K(Z)RRRGS-DCAYHKGELVWCT-NH₂ (SEQ ID NO: 67)
68) Acetyl-K(Z)EEGGS-DCAYHKGELVWCT-NH₂ (SEQ ID NO: 68)
69) Acetyl-K(Z)-(PEG)₄-DCAYHKGELVWCT-NH₂ (SEQ ID NO: 69)

R² and R³ may be a substituent in which maleimide group, DBCO group, tetrazine group, or TCO group is bound to the N-terminus or PEG of a peptide described below, or may be a substituent in which NH₂ group is bound to the C-terminus.
70) RRRGS-DCAYHKGELVWCT (SEQ ID NO: 70)
71) EEGGS-DCAYHKGELVWCT (SEQ ID NO: 71)
(PEG)₄-DCAYHKGELVWCT (SEQ ID NO: 69)

(ii) A substituent containing an IgG-BP represented by Formula (PII) described below or a substituent containing an IgG-BP consisting of an amino acid sequence in which one or several amino acids are added, deleted and/or substituted at a position other than X⁹ to X¹⁴ in the amino acid sequence of (PII): 
X⁹₁₋₂NMQX¹⁰QX¹⁴RFYEALHDPNLNEEQRNAX¹¹IX¹²SIRDDX¹³-
(Linker2)-CONH₂ ••• (PII)
 wherein (Linker2) is (GSGGS)₁₋₃, (SGSGS)₁₋₃, or (PEG)₂₋₁₀-Lys (preferably (PEG)₄-Lys), SGSGSK, SRRCR, SRRK(Z)R, SRRCRRCRRC, SRRK(Z)RRK(Z)RRK(Z), or (PEG)₁₋₈-Lys (preferably (PEG)₄-Lys), or is absent,
X⁹₁₋₂ is selected from the group consisting of GF, AF, VF, LF, IF, MF, PF, FF, WF, KF, Orn-F, CF, DF, EF, beta alanine-F, 2-aminosuberic acid-F, Dpr-F, NH₂-(PEG)n-CO (wherein n = 1-50)-F, F, K, Orn, C, D, E, 2-aminosuberic acid, Dpr, and Acetyl-K,
X¹⁰ is C or Q,
X¹¹ and X¹² are each independently selected from the group consisting of R, H, D, E, S, T, N, Q, Y, and C,
X¹³ is C or P or absent, and
X¹⁴ is R, C, K, or K(Z) .

The terminal (-NH₂) of the N-terminal amino acid of Formula (PII) may be acetylated to form (CH₃-C(= O)-NH-) group. The Cys residue (C) contained in the linker may be bound to another functional molecule via maleimide group as necessary.

Preferred examples of the IgG-BP contained in the substituent represented by Formula (PII) include the following peptides.
21. X⁹ is selected from the group consisting of GF, AF, βAlaF, NH₂-(PEG)ₙ-CO (n = 2-10)-F, F, K, Orn, C, Dpr, and Acetyl-K.
22. X⁹ is selected from the group consisting of GF, F, K, and Acetyl-K.
23. X¹⁰ is Q.
24. X¹¹ and X¹² are each independently selected from the group consisting of R, H, and E.
25. X¹¹ is R.
26. X¹² is R or K(Z) (Z is preferably azide).

More specific examples of the substituent represented by Formula (PII) include the following substituents (note that a functional group may be bound to the contained Lys residue as necessary).
72) FNMQCQRRFYEALHDPNLNEEQRNARIRSIRDDC (SEQ ID NO: 72)
73) GFNMQCQRRFYEALHDPNLNEEQRNARIRSIRDDC (SEQ ID NO: 73)
74) KNMQCQRRFYEALHDPNLNEEQRNARIRSIRDDC (SEQ ID NO: 74)
75) GFNMQCQKRFYEALHDPNLNEEQRNARIRSIRDDC (SEQ ID NO: 75)
76) KNMQCQKRFYEALHDPNLNEEQRNARIRSIRDDC (SEQ ID NO: 76)
77) FNMQQQRRFYEALHDPNLNEEQRNARIRSIRDD (SEQ ID NO: 77)
78) GKNMQCQRRFYEALHDPNLNEEQRNARIRSIRDDC (SEQ ID NO: 78) 
79) Acetyl-FNMQQQRRFYEALHDPNLNEEQRNARIRSIRDDP-
SGSGSK-NH2 (SEQ ID NO: 79)
 
80) Acetyl-FNMQCQRRFYEALHDPNLNEEQRNARIRSIRDDC-
SGSGSK-NH2 (SEQ ID NO: 80)
 
81) Acetyl-FNMQQQRRFYEALHDPNLNEEQRNARIRSIRDDP-
(PEG)4-Lys-NH2 (SEQ ID NO: 81)
 
72-2) Acetyl-FNMQCQRRFYEALHDPNLNEEQRNARIRSIRDDC-
(PEG)4-Lys-NH2 (SEQ ID NO: 72)
82) FNMQQQCRFYEALHDPNLNEEQRNARIRSIRDD-NH₂ (SEQ ID NO: 82)
83) FNMQQQRRFYEALHDPNLNEEQRNARIRSIRDDC-NH₂ (SEQ ID NO: 83)
84) FNMQQQRRFYEALHDPNLNEEQRNARIRSIRDDP-SRRK(Z)R-NH₂ (SEQ ID NO: 84)
85) FNMQQQRRFYEALHDPNLNEEQRNARIRSIRDDP-SRRCR-NH₂ (SEQ ID NO: 85)
86) FNMQQQCRFYEALHDPNLNEEQRNARICSIRDDP-SRRCRRCRRC-NH₂ (SEQ ID NO: 86) 
87) FNMQQQK(Z)RFYEALHDPNLNEEQRNARIK(Z)SIRDDP-
SRRK(Z)RRK(Z)RRK(Z)-NH2 (SEQ ID NO: 87)
88) Acetyl-KNMQQQCRFYEALHDPNLNEEQRNARIRSIRDD-NH₂ (SEQ ID NO: 88)
89) Acetyl-KNMQQQRRFYEALHDPNLNEEQRNARIRSIRDDC-NH₂ (SEQ ID NO: 89) 
90) Acetyl-KNMQQQRRFYEALHDPNLNEEQRNARIRSIRDDP-

SRRK(Z)R-NH2 (SEQ ID NO: 90)
 
91) Acetyl-KNMQQQRRFYEALHDPNLNEEQRNARIRSIRDDP-SRRCR-
NH2 (SEQ ID NO: 91)
 
92) Acetyl-KNMQQCRFYEALHDPNLNEEQRNARICSIRDDP-

SRRCRRCRRC-NH2 (SEQ ID NO: 92)
 
93) Acetyl-KNMQQQK(Z)RFYEALHDPNLNEEQRNARIK(Z)SIRDDP-

SRRK(Z)RRK(Z)RRK(Z)-NH2 (SEQ ID NO: 93)
94) GFNMQQQCRFYEALHDPNLNEEQRNARIRSIRDD-NH₂ (SEQ ID NO: 94)
95) GFNMQQQRRFYEALHDPNLNEEQRNARIRSIRDDC-NH₂ (SEQ ID NO: 95)
96) GFNMQQQRRFYEALHDPNLNEEQRNARIRSIRDDP-SRRK(Z)R-NH₂ (SEQ ID NO: 96)
97) GFNMQQQRRFYEALHDPNLNEEQRNARIRSIRDDP-SRRCR-NH₂ (SEQ ID NO: 97) 
98) GFNMQQQCRFYEALHDPNLNEEQRNARICSIRDDP-SRRCRRCRRC-

NH2 (SEQ ID NO: 98)
 
99) GFNMQQQK(Z)RFYEALHDPNLNEEQRNARIK(Z)SIRDDP-
SRRK(Z)RRK(Z)RRK(Z)-NH2 (SEQ ID NO: 99)
100) FNMQCQZRFYEALHDPNLNEEQRNARIRSIRDDC-NH₂ SEQ ID NO: 100)
101) Acetyl-KNMQCQZRFYEALHDPNLNEEQRNARIRSIRDDC-NH₂ (SEQ ID NO: 101) 
102) GFNMQCQK(Z)RFYEALHDPNLNEEQRNARIRSIRDDC-
SRRK(Z)R-NH2 (SEQ ID NO: 102)
103) FNMQCQK(Z)RFYEALHDPNLNEEQRNARIRSIRDDC-NH₂ (SEQ ID NO: 103)

104) Acetyl-KNMQCQK(Z)RFYEALHDPNLNEEQRNARIRSIRDDC-

SRRK(Z)R-NH2 (SEQ ID NO: 104)

105) GFNMQCQK(Z)RFYEALHDPNLNEEQRNARIRSIRDDC-
SRRK(Z)RRK(Z)RRK(Z)-NH2 (SEQ ID NO: 105)

106) Acetyl-KNMQCQK(Z)RFYEALHDPNLNEEQRNARIRSIRDDC-

SRRK(Z)RRK(Z)RRK(Z)-NH2 (SEQ ID NO: 106)

107) GFNMQCQK(Z)RFYEALHDPNLNEEQRNARIRSIRDDC-
SRRK(Z)RRK(Z)RRK(Z)-NH2 (SEQ ID NO: 107)

(iii) Peptides described below
108) CAWHLGELVWC (SEQ ID NO: 108)
109) DCAWHLGELVWCT (SEQ ID NO: 109)
110) DCAWHLGELVFCT (SEQ ID NO: 110)
111) DCAWHLGELVX¹⁵CT (SEQ ID NO: 111)
X¹⁵ = 1-naphtoyl, 2-naphtoyl, benzyl, or benzothiophene
112) CDCAWHLGELVWCTC (SEQ ID NO: 112)
113) CAYHLGELVWC (SEQ ID NO: 113)
114) DCAYHLGELVWCTF(2-Pya) (SEQ ID NO: 114)
115) Acetyl-(Lys[Azide]RRRGSGPDCAYHKGELVWCTFH-CONH₂) (SEQ ID NO: 115)

Considering the steric structure of the IgG-BP and the IgG, the amino acid sequence of the IgG-BP in a case where R³ has the IgG-BP may be an amino acid sequence shown in any one of SEQ ID NOs: 116 to 151 in which the N-terminal amino acid residue of an amino acid sequence shown in SEQ ID NOs: 72 to 107 is deleted.

R² may be bound to the carbon atom of the carbonyl group in Formula I or the linker via the Lys residue contained in the IgG-BP, particularly a side chain of X⁵, and R³ may be bound to the carbon atom of the benzene ring in Formula I or the linker via the Lys residue contained in the IgG-BP, particularly the above-described side chain of X5.

In a case where R² is, for example, 79), 80), 81), 72-2), 88), 89), 90), 91), 92), 93), 101), 104), or 106) described above, a side chain of any Lys residue may be bound to the carbon atom of the carbonyl group in Formula I or the linker. Similarly, in a case where R³ is 79), 80), 81), 72-2), 88), 89), 90), 91), 92), 93), 101), 104), or 106) described above, a side chain of any Lys residue may be bound to the carbon atom of the benzene ring in Formula I or the linker.

In the above-described IgG-BP, any two cysteine residues may form an intramolecular bond in the peptide via a disulfide bond.

R⁴ is H or a substituted or unsubstituted alkyl group having 1 to 8 carbon atoms. R⁴ is, for example, an alkyl group having 1 to 6, 1 to 4, or 1 to 3 carbon atoms, preferably ethyl group, and more preferably methyl group.

The salt of the cyclic peptide according to the present embodiment is not particularly limited as long as it is a pharmacologically acceptable salt, and may be either an acidic salt or a basic salt. Examples of the salt include alkali metal salts such as lithium salts, sodium salts, and potassium salts, alkaline earth metal salts such as magnesium salts and calcium salts, inorganic acid salts such as hydrochloride salts, hydrobromide salts, sulfate salts, nitrate salts, oxalate salts, and phosphate salts, and organic acid salts such as acetate salts, propionate salts, hexanoate salts, cyclopentane propionate salts, glycolate salts, pyruvate salts, lactate salts, malonate salts, succinate salts, malate salts, fumarate salts, tartrate salts, citrate salts, benzoate salts, o-(4-hydroxybenzoyl)benzoate salts, cinnamate salts, mandelate salts, methanesulfonate salts, ethanesulfonate salts, 1,2-ethanedisulfonate salts, 2-hydroxyethanesulfonate salts, benzenesulfonate salts, p-chlorobenzenesulfonate salts, 2-naphthalenesulfonate salts, p-toluenesulfonate salts, camphorsulfonate salts, glucoheptanoate salts, 3-phenylpropionate salts, trimethyl acetate salts, tertiary butyl acetate salts, lauryl sulfate salts, gluconate salts, glutamate salts, hydroxynaphthoate salts, salicylate salts, stearate salts, trifluoroacetic acid (TFA) salts, maleate salts, and muconate salts.

The compound according to the present embodiment can be synthesized with a known method on the basis of the structure represented by Formula I described above. For example, in synthesis of a compound in which R² has the IgG-BP, amino group of a peptide on a resin synthesized with a solid-phase peptide synthesis method is glutarylated to condense N-methyl-1,2-phenylenediamine, and then treatment with 4-nitrophenyl chloroformate is to be performed. For example, in synthesis of a compound in which R³ has the IgG-BP, a MeDbz residue is inserted using Fmoc-MeDbz (3-((9-fluorenylmethoxycarbonyl)amino)-4-(methylamino)benzoic acid) in extension of a peptide on a resin, and thus the skeleton shown in Formula I described above can be constructed at the MeDbz residue site by treatment with 4-nitrophenyl chloroformate on the protected peptide resin.

The compound according to the present embodiment or a salt of the compound does not have NHS group, which is easily hydrolyzed, and therefore is chemically stable and can be stored for a long period of time. The compound or a salt of the compound has an active ester precursor represented by Formula I, and therefore the compound or the salt mixed with the IgG can specifically modify a predetermined site of the Fc region. If the IgG is modified with a reactive functional group, a drug, a labeling substance, or the like can be bound to the IgG via the reactive functional group. When the drug is attached to the IgG, an antibody drug conjugate is obtained in which the drug is site-specifically bound. Thus, the property of the drug can be imparted to the IgG, and therefore the IgG can be applied to a drug delivery system (DDS) and the like. Furthermore, a site-specific modification of the IgG with a labeling substance enables quantitative control of the labeling substance, and therefore is useful for tracking, distribution, detection, and the like of the IgG.

As an example of the compound according to the present embodiment, the structure of a compound A is shown below. The compound A has an IgG-BP in which R² has an amino acid sequence shown in SEQ ID NO: 152. In the following structure of the compound A, N, M, and Q in -N-M-Q-NH-, and RFYEALHDPNLNEEQRNARIRSIRDD represent amino acids having a one-letter code.

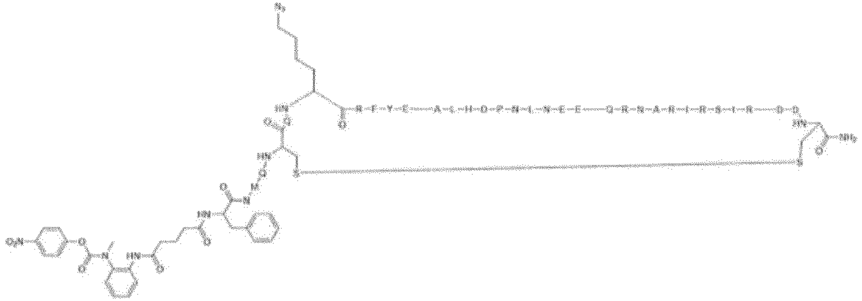

As an example of the compound according to the present embodiment, the structure of a compound B is shown below. The compound has an IgG-BP in which R³ has an amino acid sequence shown in SEQ ID NO: 153. In the following structure of the compound B, N, M, and Q in -C(=O)-N-M-Q-NH-, and QRRFYEALHDPNLNEEQRNARIRSIRDD represent amino acids having a one-letter code.

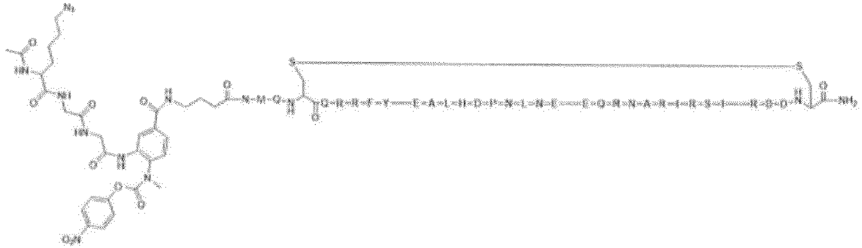

Examples of the compound in which R³ has the IgG-BP include Formulas VI to IX described below.

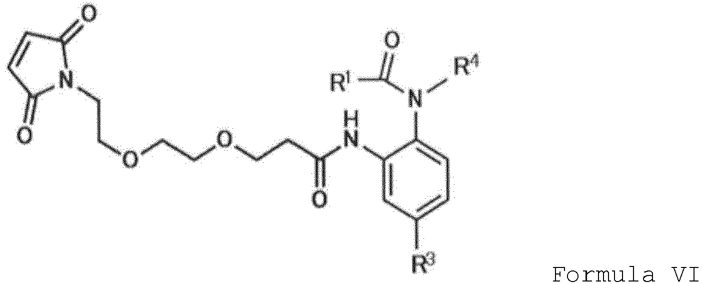

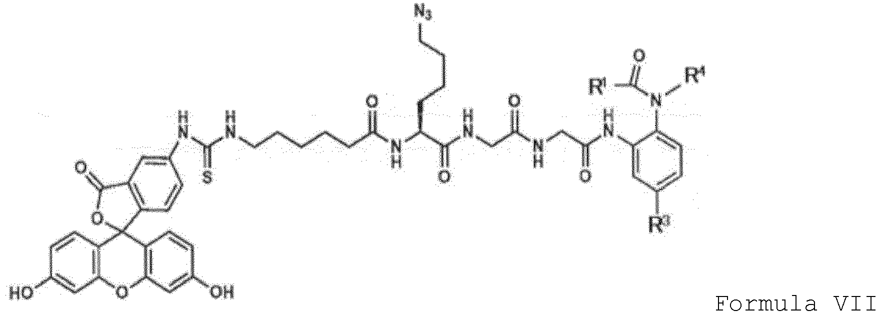

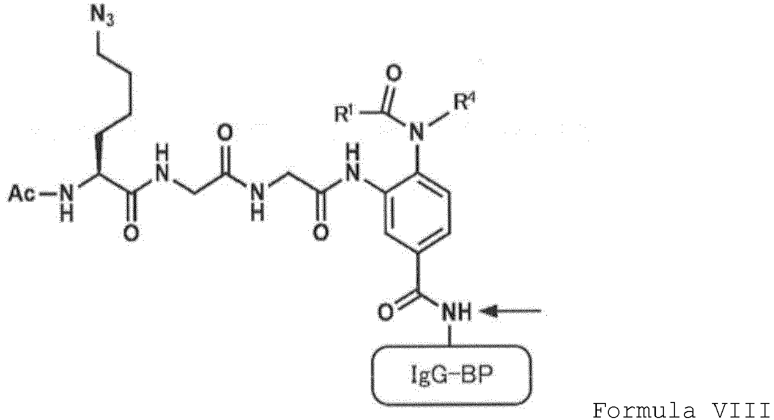

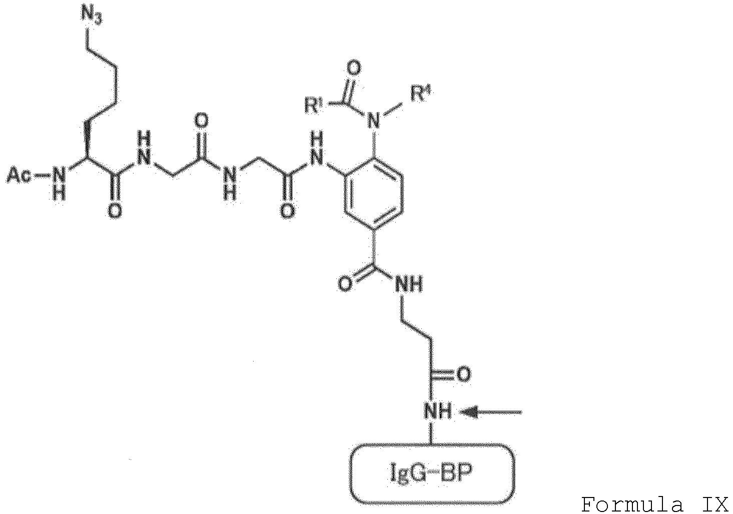

In Formulas VIII and IX, an amino acid residue that is not the N-terminus of the IgG-BP, such as amino group of a side chain of Lys residue, is bound to the carbon atom of the benzene ring in Formula I via a linker. In Formulas VIII and IX, -NH- indicated by an arrow represents amino group of a side chain of Lys residue.

Furthermore, as a compound C and a compound D in which R³ has the IgG-BP, Formulas X and XI described below are exemplified, respectively. The amino acid sequence of the IgG-BP of the compound C and the compound D is the amino acid sequence shown in SEQ ID NO: 153, which is the same as that of the IgG-BP of the above-described compound B.

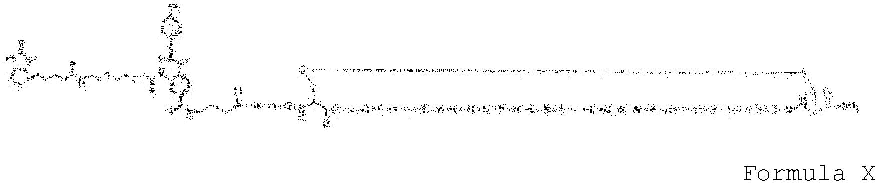

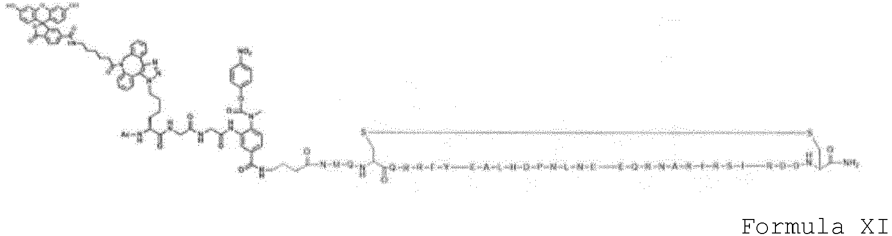

In another embodiment, an antibody modification reagent is provided that contains the compound according to the present embodiment or a salt of the compound. In another embodiment, a method for producing a modified antibody using the antibody modification reagent is provided. The method for producing a modified antibody includes a reaction step. In the reaction step, the antibody modification reagent is reacted with an IgG.

Any reaction conditions can be adopted in the reaction step as long as the antibody modification reagent is exposed to the IgG, but for example, the IgG and the antibody modification reagent are to be mixed in a buffer solution having a pH of 7.0 to 9.0. The concentrations of the IgG and the antibody modification reagent in the buffer solution are appropriately adjusted. The molar ratio of the IgG to the antibody modification reagent in the buffer solution is, for example, 1 : 1 to 10, 1 : 2 to 8, or 1 : 3 to 6, and preferably 1 : 5.

### (Modified Antibody)

The modified antibody according to the present embodiment has an atomic group containing, in Formula I described above, from the carbonyl group to which R² is bound to R². The modified antibody is preferably obtained by reacting the above-described compound or a salt of the compound with an IgG.

For example, the modified antibody comprises a drug, a reactive functional group, or a labeling substance bound to Lys of an Fc region of an IgG in a monovalent or bivalent form via an atomic group containing Lys-Gly-Gly. The IgG is not particularly limited, but is particularly preferably a human IgG, and is, for example, trastuzumab or tocilizumab.

The above-described atomic group may be, for example, a peptide chain containing Lys-Gly-Gly, or may be an atomic group in which a linker or the like is further bound to the peptide chain. The drug, the reactive functional group, or the labeling substance may be bound to the N-terminus of the peptide chain via a linker, or amino group of a side chain of Lys contained in the atomic group may be substituted with the drug, the reactive functional group, or the labeling substance. The N-terminus of Lys in Lys-Gly-Gly may be acetylated.

The following structures are examples of a structure of the modified antibody to which the drug, the reactive functional group, or the labeling substance is bound via the atomic group containing Lys-Gly-Gly. Note that -NH-indicated by an arrow represents amino group of a side chain of Lys residue.

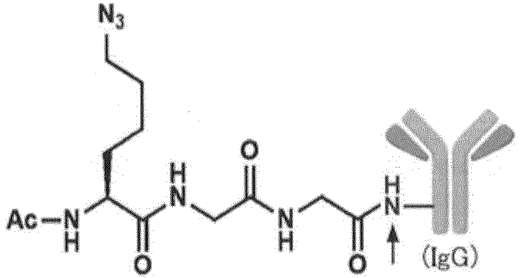

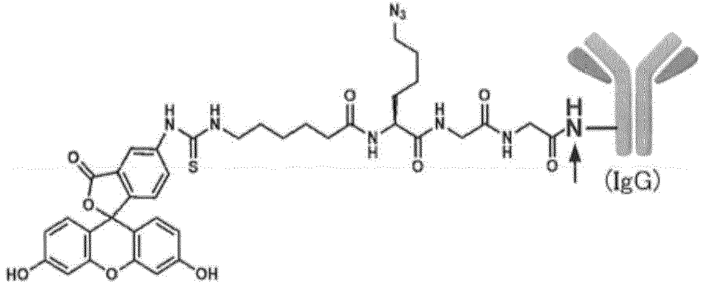

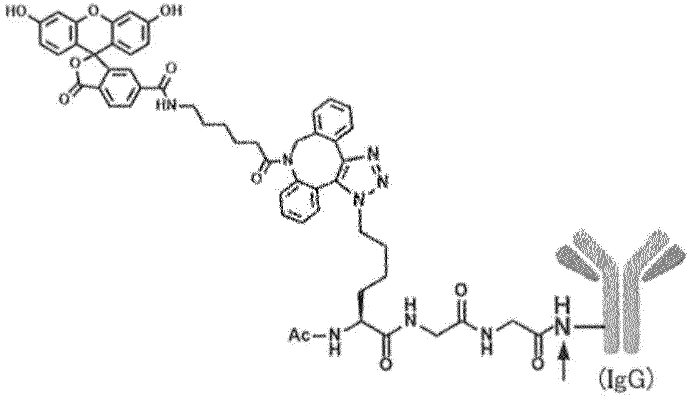

The modified antibody may comprise the above-described drug, reactive functional group, or labeling substance bound via an atomic group containing PEG, Gly, or a combination thereof bound to Lys of the Fc region of the IgG in a monovalent or bivalent form. The following structures are examples of a structure of the modified antibody. Note that -NH- indicated by an arrow represents amino group of a side chain of Lys residue.

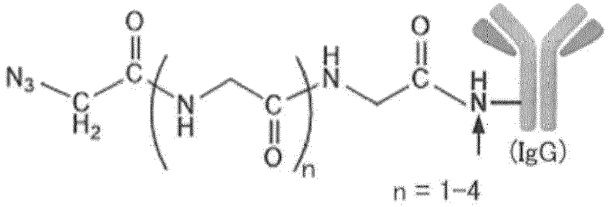

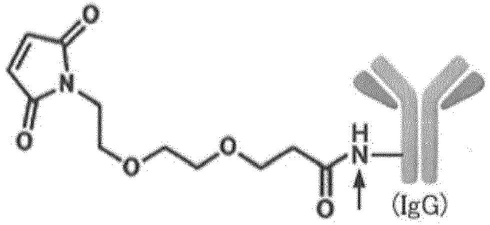

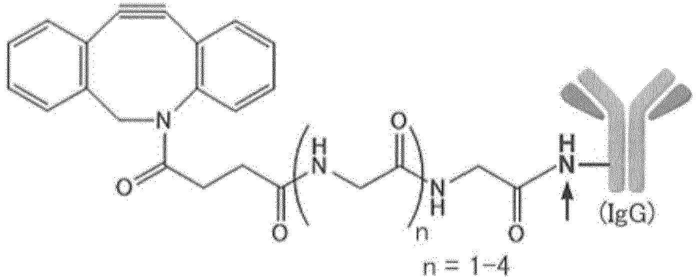

### (Additional Statement)

(Additional statement 1) A compound represented by Formula I described above or a salt of the compound.

(Additional statement 2) The compound or the salt of the compound according to Additional statement 1, wherein R¹ is nitrophenoxy group.

(Additional statement 3) The compound or the salt of the compound according to Additional statement 1 or 2, wherein R² has the above-described IgG-binding peptide, and R³ is absent.

(Additional statement 4) The compound or the salt of the compound according to any one of Additional statements 1 to 3, wherein R² is represented by Formula II described above wherein R^{2a} represents the above-described IgG-binding peptide.

(Additional statement 5) The compound or the salt of the compound according to Additional statement 3 or 4, wherein the above-described IgG-binding peptide is modified with a drug, a reactive functional group, or a labeling substance.

(Additional statement 6) The compound or the salt of the compound according to Additional statement 1 or 2, wherein R³ has the above-described IgG-binding peptide, and R² has a drug, a reactive functional group, or a labeling substance.

(Additional statement 7) The compound or the salt of the compound according to any one of Additional statements 1, 2, and 6, wherein R³ is represented by any one of Formulas III, IV, and V described above wherein R^{3a} represents the above-described IgG-binding peptide.

(Additional statement 8) The compound or the salt of the compound according to any one of Additional statements 1 to 7, wherein the above-described IgG-binding peptide has an amino acid sequence shown in any one of SEQ ID NOs: 1 to 115.

(Additional statement 9) The compound or the salt of the compound according to Additional statement 6 or 7, wherein the above-described IgG-binding peptide has an amino acid sequence shown in any one of SEQ ID NOs: 116 to 151.

(Additional statement 10) An antibody modification reagent comprising the compound or the salt of the compound according to any one of Additional statements 1 to 9.

(Additional statement 11) A method for producing a modified antibody, the method comprising a reaction step of reacting the antibody modification reagent according to Additional statement 10 with an IgG.

### Examples

The present invention will be described more specifically with reference to the following examples, but the present invention is not limited by the examples.

### Example 1: Synthesis of Compound A

The above-described compound A was synthesized as follows. A protected peptide resin was constructed using 0.25 mmol of Rink Amide PEG resin (0.55 mmol/g) with a PurePep Chorus solid-phase peptide synthesizer (manufactured by Gyros Protein Technologies) by repeating deFmoc with piperidine/NMP (1 : 4) and condensation with Fmoc protected amino acid/DIC/Oxyma (1 mmol/1 mmol/1 mmol). However, condensation of Fmoc-Lys(N₃) was performed with Fmoc-Lys (N₃)/DIC/Oxyma (0.50 mmol/0.50 mmol/0.50 mmol).

To the N-terminal amine of the obtained protected peptide resin (0.20 mmol), glutaric anhydride (0.1 g) was reacted in N-methyl-2-pyrrolidone (NMP) for 1 hour in the presence of triethylamine (35 µL). Then, to the obtained N-terminal carboxylic acid resin, N-methyl-1,2-phenylenediamine dihydrochloride (0.234 g) was condensed using coupling reagents PyAop (0.625 g), HOAt (0.163 g), and N,N-diisopropylethylamine (DIEA) (0.612 mL). Then, 4-nitrophenyl chloroformate (0.4 g) was reacted in dichloromethane for 1 hour to construct a phenoxycarbonylated N-methyl-o-diaminobenzene (PMD)(NO₃) skeleton. The obtained resin was treated with a trifluoroacetic acid (TFA) solution to perform deresination and deprotection, and solidified with diethyl ether to obtain 721 mg of a crude peptide (2SH). In acetic acid/water (1 : 1), 250 mg of the obtained crude peptide (2SH) was dissolved, and under ice cooling, 1 equivalent of a 0.1 M iodine-methanol solution was slowly added. After 90 seconds, the reaction was quenched with an aqueous ascorbic acid solution, purification was performed with a reverse phase high performance liquid chromatography (HPLC) column, and lyophilization was performed to obtain 50 mg of PMD(NO₂)-αZ34C (compound A) as a desired SS peptide.

### Example 2: Synthesis of Compound B

The above-described compound B was synthesized as follows. A protected peptide resin was constructed using 0.25 mmol of Rink Amide PEG resin (0.55 mmol/g) with a PurePep Chorus solid-phase peptide synthesizer (manufactured by Gyros Protein Technologies) by repeating deFmoc with piperidine/NMP (1 : 4) and condensation with Fmoc protected amino acid/DIC/Oxyma (1 mmol/1 mmol/1 mmol). However, condensation of Fmoc-MeDbz was performed with Fmoc-MeDbz/HCTU/6-Cl HOBt/DIEA (1 mmol/1 mmol/1 mmol/2 mmol), and condensation of Fmoc-Lys(N₃) was performed with Fmoc-Lys(N₃)/DIC/HOAt (0.375 mmol/0.375 mmol/0.375 mmol).

To the obtained protected peptide resin (0.25 mmol), 4-nitrophenyl chloroformate (0.5 g) was reacted in dichloromethane for 1 hour to construct a PMD(NO₂) skeleton. The obtained resin was treated with a TFA solution to perform deresination and deprotection, and solidified with diethyl ether to obtain 966 mg of a crude peptide (2SH). The obtained crude peptide was purified with a reverse phase HPLC column and lyophilized to obtain 147 mg of a 2SH peptide. Then, the crude peptide (2SH) was dissolved in acetic acid/water (1 : 1), and under ice cooling, 1 equivalent of a 0.1 M iodine-methanol solution was slowly added. After 30 seconds, the reaction was quenched with an aqueous ascorbic acid solution, purification was performed with a reverse phase HPLC column, and lyophilization was performed to obtain 87 mg of AzideKGG-PMD(NO₂)-αZ34C(F1Gaba,K7R) (compound B) as a desired SS peptide.

For comparison, the following compound with DSG was synthesized.

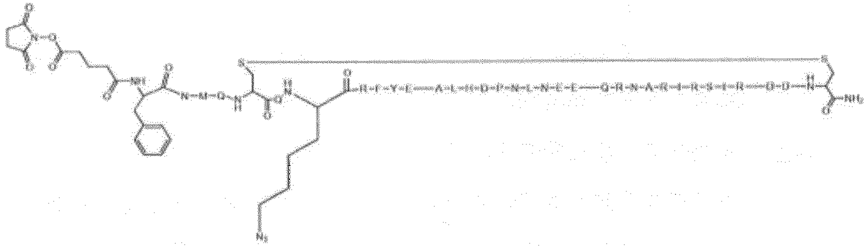

A protected peptide resin was constructed using 0.25 mmol of Rink Amide PEG resin (0.55 mmol/g) with a PurePep Chorus solid-phase peptide synthesizer (manufactured by Gyros Protein Technologies) by repeating deFmoc with piperidine/NMP (1 : 4) and condensation with Fmoc protected amino acid/DIC/Oxyma (1 mmol/1 mmol/1 mmol). However, condensation of Fmoc-Lys(N₃) was performed with Fmoc-Lys(N₃)/DIC/Oxyma (0.50 mmol/0.50 mmol/0.50 mmol).

The obtained protected peptide resin was treated with a TFA solution to perform deresination and deprotection, and the crude peptide (2SH) was solidified with diethyl ether and collected by filtration. The obtained crude peptide (2SH) was dissolved in acetic acid/water (1 : 1), and under ice cooling, a 0.1 M iodine-methanol solution was slowly added. After 60 seconds, the reaction was quenched with an aqueous ascorbic acid solution, purification was performed with a reverse phase HPLC column, and lyophilization was performed to obtain 61 mg of a precursor peptide (SS) having an N-terminal free amine component. Finally, 60 mg of the precursor peptide (SS) was dissolved in DMSO, and DSG (18 mg) and DIEA (12 µL) were slowly added to convert the N-terminal amino group to succinimidyl ester for activation. After 10 minutes, the mixture was diluted with 0.1% TFA water, purified with a reverse phase HPLC column, and lyophilized to obtain 19 mg of desired DSG-αZ34C (hereinafter, referred to as comparative example).

### Test Example 1: Examination of Stability

The compound A, the compound B, or the comparative example was dissolved in DMSO at a content of 20 mg/mL, allowed to stand at room temperature or -20°C for a predetermined time, and analyzed by HPLC.

### (Results)

For the compound A, the compound B, and the comparative example, the ratio of the area of the main peak to the sum of the areas of all peaks (HPLC purity) is shown in Table 1. The compound A and the compound B were stabler than the comparative example at room temperature and -20°C.

| | Room temperature | | -20°C | |
|---|---|---|---|---|
| | 0 Hours | 22 Hours | 0 Days | 30 Days |
| Comparative example | 85% | 83% | 92% | 85% |
| Compound A | 94% | 93% | 94% | 94% |
| Compound B | 98% | 92% | 98% | 98% |

### Example 3: Evaluation of Reaction of Compound A and Compound B to human IgG

### Reaction with Human IgG1

The compound A or the compound B was reacted with an antibody (human IgG1 antibody drug ACTEMRA, tocilizumab) under four pH conditions. That is, 20 µL of a 0.5 M acetate buffer solution (pH 5.5), a 0.5 M phosphate buffer solution (pH 7.0), a 0.5 M Hepes buffer solution (pH 8.0), or a 0.5 M bicarbonate buffer solution (pH 8.9) was added to 20 µL of a 20 mg/ml IgG solution, and 138.4 µL of distilled water was added. Finally, 1.56 µL of the 10 mM compound A or 10 mM compound B dissolved in DMSO was added, the mixture was rapidly stirred, and then the total amount was set to 200 µL (the IgG concentration in the final reaction product was 15.6 µM, and the molar ratio of the reagent was 5, that is, the concentration was 78 µM). After 2 hours, the mixture was sampled, the reaction was quenched with formic acid at a final concentration of 10%, and then the sample was analyzed by LC-MS.

### LC-MS Analysis of Reaction Product

The reaction solution after stopping the reaction was diluted 5 times with 0.1% formic acid, and 20 µL of the diluted solution was analyzed with a BioAccord LC-MS system (manufactured by Waters Corporation) to which a Protein BEH C4 column (300 Å, 1.7 µm, 2.1 × 500 mm, manufactured by Waters Corporation) is connected (flow rate: 0.4 mL/min, elution: linear gradient from 1% CH₃CN to 50% CH₃CN with 0.1% formic acid, column temperature: 80°C).

### (Results)

Figs. 3 and 4 show the results of LC-MS of the reaction solution of the compound A and the IgG after 2 hours. (A), (B), (C), and (D) of Fig. 3 are elution chromatograms (280 nm) of the reaction solutions at pH 5.5, 7.0, 8.0, and 8.9, respectively. A peak at 2.23 minutes or 2.25 minutes is a peak corresponding to elution of the IgG. (A), (B), (C), and (D) of Fig. 4 show results of mass spectrometry of peaks of the IgG in the reaction solutions at pH 5.5, 7.0, 8.0, and 8.9, respectively. Molecular species with masses of 147879 Da, 148041 Da, and 148203 Da in the reaction solution at pH 5.5 shown in Fig. 4(A) are N-G0F × 2 (a molecular species 1), N-G0F × 1 + N-G1F × 1 (a molecular species 2), and N-G1F × 2 (a molecular species 3), respectively, detected by diversity of sugar chains of the IgG. No molecular species modified by addition of a peptide was observed at pH 5.5.

Also in the reaction solution at pH 7.0 shown in Fig. 4(B) and the reaction solution at pH 8.0 shown in Fig. 4(C), no molecular species modified was observed in almost the same manner as in the reaction solution at pH 5.5. Meanwhile, in the reaction solution at pH 8.9 shown in Fig. 4(D), molecular species with masses of 152195 Da, 152357 Da, and 152519 Da were observed. These are increased by 4316 Da, 4316 Da, and 4317 Da from the masses of the molecular species 1, 2, and 3 of the IgG, respectively. This increase almost matched the mass 4333 Da expected as an increase at the time of coupling of the peptide to the IgG with the compound A, and thus it was shown that the compound A was reacted with the IgG at pH 8.9.

Figs. 5 and 6 show the results of LC-MS of the reaction solution of the compound B and the IgG after 2 hours. (A), (B), (C), and (D) of Fig. 5 are elution chromatograms (280 nm) of the reaction solutions at pH 5.5, 7.0, 8.0, and 8.9, respectively. A peak at 1.93 minutes or 2.02 minutes is a peak corresponding to elution of the compound B. A peak at 2.25 minutes or 2.27 minutes is a peak corresponding to elution of the IgG. (A), (B), (C), and (D) of Fig. 6 show results of mass spectrometry of peaks of the IgG in the reaction solutions at pH 5.5, 7.0, 8.0, and 8.9, respectively. As shown in Fig. 6(A), at pH 5.5, the molecular species 1 (147882 Da), the molecular species 2 (148044 Da), and the molecular species 3 (148208 Da) of the IgG were observed, but at pH 7.0 shown in Fig. 6(B), the molecular species 1 was decreased, and a molecular species with a mass of 148193 Da was increased. This difference of 311 Da matched the mass of azidated KGG coupled by a reaction of the compound B (311.33 Da).

Furthermore, at pH 8.0 shown in Fig. 6(C) and pH 8.9 shown in Fig. 6(D), molecular species with a mass of 148505 Da and 148501 Da were increased, respectively. The mass of these molecular species was increased by 623 Da or 619 Da from the mass of the molecular species 1 and corresponded to 2 times the mass of azidated KGG added (311.33), and therefore two of azidated KGG were considered to be added to the IgG. The molecular species 2 was also increased in mass, at pH 7.0, to be a molecular species with a mass of 148353 Da in which one of azidated KGG was considered to be added and a molecular species with a mass of 148664 Da in which two of azidated KGG were considered to be added, and at pH 8.0 and 8.9, subsequent addition from one of azidated KGG to two of azidated KGG was also observed. From the above, it was shown that the compound B was reacted with the IgG at a pH of 7.0 or more.

### Example 4: Evaluation of Reaction of Compound B to Mouse IgG

### Reaction with Mouse IgG

The compound B and a mouse IgG2 antibody (anti-DYKDDDDK mouse IgG2b monoclonal antibody) were reacted under two pH conditions as follows. To 30 µL of a 0.98 mg/mL IgG solution, 22 µL of a 0.2 M Hepes buffer solution (pH 8.0) or a 0.2 M bicarbonate buffer solution (pH 8.9) was added. Finally, 8 µL of the 50 µM compound B dissolved in DMSO was added, the mixture was rapidly stirred, and then the total amount was set to 60 µL (the IgG concentration in the final reaction product was 1.67 µM, and the molar ratio of the reagent was 4, that is, the concentration was 6.67 µM). After 2 hours, the mixture was sampled, the reaction was stopped with formic acid at a final concentration of 5%, and then the sample was analyzed by LC-MS in the same manner as in Example 3.

### (Results)

Figs. 7 and 8 show the results of LC-MS of the reaction solution of the compound B and the mouse IgG. (A), (B), and (C) of Fig. 7 are elution chromatograms (280nm) of an antibody solution having a pH of 8.0 before addition of the compound B, a solution having a pH of 8.0 2 hours after reaction, and a solution having a pH of 8.9 2 hours after reaction, respectively. The peaks at 2.30 minutes and 2.53 minutes are peaks corresponding to elution of the mouse IgG. (A), (B), and (C) of Fig. 8 show results of mass spectrometry of peaks of the IgG in the antibody solution having a pH of 8.0 before addition of the compound B, the solution having a pH of 8.0 2 hours after reaction, and the solution having a pH of 8.9 2 hours after reaction, respectively. Molecular species with masses of 150094 Da, 150256 Da, and 150419 Da in the solution at pH 8.0 before addition of the compound B, shown in Fig. 8(A), are N-G0F × 2 (a molecular species 1), N-G0F × 1 + N-G1F × 1 (a molecular species 2), and N-G1F × 2 (a molecular species 3), respectively, detected by diversity of sugar chains of the IgG.

As shown in Figs. 8(B) and 8(C), at pH 8.0 and pH 8.9, the molecular species 1 to 3 were decreased, while molecular species with masses of 150569 Da, 150729 Da, 150884 Da, and 151039 Da were newly increased. The mass of 150569 Da is an increase of 313 Da from the mass of the molecular species 2. The mass of 150729 Da is an increase of 310 Da from the mass of the molecular species 3 and an increase of 635 Da from the mass of the molecular species 1. The mass of 150884 Da is an increase of 628 Da from the mass of the molecular species 2. The mass of 151039 Da is an increase of 620 Da from the mass of the molecular species 3. These differences in mass correspond to the mass of the azidated KGG coupled to the antibody by the reaction of the compound B (311.33 Da) or 2 times of the mass, and thus coupling of the first azidated KGG followed by coupling of the second azidated KGG was observed. From the above, it was shown that the compound B was also reacted with the mouse antibody at a pH of 8.0 or more.

### Example 5: Evaluation of Reaction of Compound B to Rabbit IgG

### Reaction with Rabbit IgG

The compound B and a rabbit antibody (anti-mouse IgG1 monoclonal rabbit antibody) were reacted under the condition of pH 8.0 as follows. To 25 µL of a 1.93 mg/mL IgG solution, 10 µL of a 0.5 M HEPES buffer solution (pH 8.0) and 5 µL of distilled water were added. Finally, 60 µL of the 89.3 µM compound B dissolved in distilled water was added, the mixture was rapidly stirred, and then the total amount was set to 100 µL (the IgG concentration in the final reaction product was 6.7 µM, and the molar ratio of the compound B was 8, that is, the concentration was 53.6 µM). After 2 hours, the mixture was sampled, the reaction was stopped with formic acid at a final concentration of 5%, and then the sample was analyzed by LC-MS in the same manner as in Example 3.

### (Results)

Figs. 9 and 10 show the results of LC-MS of the reaction solution of the compound B and the rabbit IgG. (A) and (B) of Fig. 9 are elution chromatograms (280 nm) of an antibody solution having a pH of 8.0 before addition of the compound B and a solution having a pH of 8.0 24 hours after reaction, respectively. (A) and (B) of Fig. 10 show results of mass spectrometry of peaks of the IgG in the antibody solution having a pH of 8.0 before addition of the compound B and the solution having a pH of 8.0 24 hours after reaction, respectively. Molecular species with masses of 144237 Da, 144397 Da, and 144559 Da in the reaction solution at pH 8.0 before addition of the compound B, shown in Fig. 10(A), are N-G0F × 2 (a molecular species 1), N-G0F × 1 + N-G1F × 1 (a molecular species 2), and N-G1F × 2 (a molecular species 3), respectively, detected by diversity of sugar chains of the IgG.

As shown in Fig. 10(B), the molecular species 1 to 3 were decreased, while molecular species with masses of 144544 Da, 144713 Da, 144864 Da, 145019 Da, and 145179 Da were newly increased. The mass of 144544 D is an increase of 307 Da from the mass of the molecular species 1. The mass of 144713 Da is an increase of 316 Da from the mass of the molecular species 2. The mass of 144864 Da is an increase of 305 Da from the mass of the molecular species 3 and an increase of 627 Da from the mass of the molecular species 1. The mass of 145019 Da is an increase of 622 Da from the mass of the molecular species 2. The mass of 1445179 Da is an increase of 620 Da from the mass of the molecular species 3. These differences in mass correspond to the mass of the azidated KGG coupled by the reaction of the compound B (311.33 Da) or 2 times of the mass, and thus coupling of the first azidated KGG followed by coupling of the second azidated KGG was observed. From the above, it was shown that the compound B was also reacted with the rabbit antibody at a pH of 8.0 or more.

### Example 6: Synthesis of Compound C

The above-described compound C was synthesized as follows. A protected peptide resin having N-terminal MeDbz was constructed using 0.25 mmol of Rink Amide PEG resin (0.48 mmol/g) with a PurePep Chorus solid-phase peptide synthesizer (manufactured by Gyros Protein Technologies) by repeating deFmoc with piperidine/NMP (1 : 4) and condensation with Fmoc protected amino acid/DIC/Oxyma (1 mmol/1 mmol/1 mmol). Then, Fmoc-AEEA (aminoethoxyethoxyacetic acid) and biotin were sequentially condensed using 0.03 mmol of the protected peptide resin. However, condensation of Fmoc-MeDbz was performed with Fmoc-MeDbz/HCTU/6-Cl HOBt/DIEA (1 mmol/1 mmol/1 mmol/2 mmol), condensation of Fmoc-AEEA was performed with Fmoc-AEEA/DIC/Oxyma (1 mmol/1 mmol/1 mmol), and condensation of biotin was performed with biotin/DIC/Oxyma (0.5 mmol/0.5 mmol/0.5 mmol).

To the obtained protected peptide resin (0.03 mmol), 4-nitrophenyl chloroformate (0.06 g) was reacted in dichloromethane for 1 hour to construct a PMD(NO₂) skeleton. The obtained resin was treated with a TFA solution to perform deresination and deprotection, and solidified with diethyl ether to obtain 130 mg of a crude peptide (2SH). Then, 65 mg of the crude peptide (2SH) was dissolved in acetic acid/water (1 : 1), and under ice cooling, several equivalents of a 0.1 M iodine-methanol solution ware slowly added. After 60 seconds, the reaction was quenched with an aqueous ascorbic acid solution, purification was performed with a reverse phase HPLC column, and lyophilization was performed to obtain 10 mg of biotin-AEEA-PMD(NO₂)-aZ34C (F1Gaba,K7R) (compound C) as a desired SS peptide.

### Example 7: Synthesis of Compound D

The above-described compound D was synthesized as follows. In 10 µL of DMSO, 0.62 mg of the compound B was dissolved, 0.15 mg of FAM-DBCO was added at a molar ratio of 2, and the mixture was allowed to stand (the concentration of the compound D in the final reaction product was 10.8 mM). After 1 hour, high performance liquid chromatography was used to confirm disappearance of the compound B and formation of the compound D. The compound D was directly used for reaction with an IgG without purification.

### Example 8: Evaluation of Reaction of Compound C to Human IgG

### Reaction with Human IgG

The compound C and an antibody (tocilizumab) were reacted under four pH conditions. That is, 40 µL of a 0.5 M acetate buffer solution (pH 5.5), a 0.5 M phosphate buffer solution (pH 7.0), a 0.5 M HEPES buffer solution (pH 8.0), or a 0.5 M bicarbonate buffer solution (pH 8.9) was added to 20 µL of a 20 mg/ml IgG solution, and 138.4 µL of distilled water was further added. Finally, 1.56 µL of the 10 mM compound C dissolved in DMSO was added, the mixture was rapidly stirred, and then the total amount was set to 200 µL (the IgG concentration in the final reaction product was 15.6 µM, and the molar ratio of the compound C was 5, that is, the concentration was 78 µM). After 2 hours, the mixture was sampled, the reaction was stopped with formic acid at a final concentration of 10%, and then the sample was analyzed by LC-MS in the same manner as in Example 3.

### (Results)

Figs. 11 and 12 show the results of LC-MS of the reaction solution of the compound C and the human IgG 2 hours after the reaction. (A), (B), (C), and (D) of Fig. 11 are elution chromatograms (280 nm) of the reaction solutions at pH 5.5, 7.0, 8.0, and 8.9, respectively. A peak at 2.27 minutes is a peak corresponding to elution of the IgG. (A), (B), (C), and (D) of Fig. 12 show results of mass spectrometry of peaks of the IgG in the reaction solutions at pH 5.5, 7.0, 8.0, and 8.9, respectively. Molecular species with masses of 147878 Da, 148039 Da, and 148201 Da in the reaction solution at pH 5.5 shown in Fig. 12(A) are N-G0F × 2 (a molecular species 1), N-G0F × 1 + N-G1F × 1 (a molecular species 2), and N-G1F × 2 (a molecular species 3), respectively, detected by diversity of sugar chains of the IgG.

As shown in Fig. 12(A), no molecular species modified by the compound C was observed at pH 5.5. Meanwhile, at pH 7.0 shown in Fig. 12(B), the molecular species 1 was decreased, and the molecular species with a mass of 148248 Da was increased. This difference of 370 Da matched the mass of biotin-AEEA (hereinafter, also simply referred to as "biotin-PEG") added by a reaction of the compound C (372.46 Da).

Furthermore, at pH 8.0 shown in Fig. 12(C) and pH 8.9 shown in Fig. 12(D), molecular species with a mass of 148620 Da and 148619 Da were increased, respectively. The masses of these molecular species are increased by 742 Da and 743 Da, respectively, from the mass of the molecular species 1. These differences in mass corresponded to 2 times the mass of biotin-PEG coupled (372.46 Da), and therefore two of biotin-PEG were considered to be coupled to the IgG. The molecular species 2 was also increased in mass, at pH 7.0, to be a molecular species with a mass of 148411 Da in which one of biotin-PEG was considered to be added, and at pH 8.0 and 8.9, to be a molecular species with a mass of 148409 Da in which one of biotin-PEG was considered to be added and a molecular species with a mass of 148782 Da in which two of biotin-PEG were considered to be added, and subsequent addition from one of biotin-PEG to two of biotin-PEG was also observed. From the above, it was shown that the compound C was reacted with the human IgG at a pH of 7.0 or more.

### Example 9: Evaluation of Reaction of Compound D to Humanized IgG

### Reaction with IgG

The compound D and an antibody (anti-HER2 humanized monoclonal antibody drug Herceptin, trastuzumab) were reacted under a condition of pH 8.0 or 8.9 as follows. To 20 µL of a 20 mg/ml IgG solution, 40 µL of a 0.5 M HEPES buffer solution (pH 8.0) or a 0.5 M bicarbonate buffer solution (pH 8.9) was added, and 138 µL of distilled water was further added. Finally, 2 µL of a 10.8 mM DMSO solution of the compound D was added, the mixture was rapidly stirred, and then the total amount was set to 100 µL (the IgG concentration in the final reaction product was 13.5 µM, and the molar ratio of the compound D was 8, that is, the concentration was 108 µM). After 24 hours, the mixture was sampled, the reaction was stopped with formic acid at a final concentration of 5%, and then the sample was analyzed by LC-MS in the same manner as in Example 3.

### (Results)

Figs. 13 and 14 show the results of LC-MS of the reaction solution of the compound D and the humanized IgG. (A), (B), and (C) of Fig. 13 are elution chromatograms (280nm) of an antibody solution before addition of the compound D, a solution having a pH of 8.0 24 hours after reaction, and a solution having a pH of 8.9 24 hours after reaction, respectively. A peak at 2.23 minutes is a peak corresponding to elution of the IgG. (A), (B), and (C) of Fig. 14 show results of mass spectrometry of peaks of the IgG in the antibody solution before addition of the compound D, the solution having a pH of 8.0 24 hours after reaction, and the solution having a pH of 8.9 24 hours after reaction, respectively. Molecular species with masses of 148058 Da, 148220 Da, and 148381 Da in the reaction solution at pH 8.0 before addition of the compound D, shown in Fig. 14(A), are N-G0F × 2 (a molecular species 1), N-G0F × 1 + N-G1F × 1 (a molecular species 2), and N-G1F × 2 (a molecular species 3), respectively, detected by diversity of sugar chains of the IgG.

As shown in Fig. 14(B), at pH 8.0, the molecular species 1 to 3 were decreased, while molecular species with masses of 149048 Da, 149209 Da, 149371 Da, 150036 Da, 150196 Da, and 150356 Da were newly increased. The mass of 149048 Da is an increase of 990 Da from the mass of the molecular species 1. The mass of 149209 Da is an increase of 989 Da from the mass of the molecular species 2. The mass of 149371 Da is an increase of 990 Da from the mass of the molecular species 3. The mass of 150036 Da is an increase of 1978 Da from the mass of the molecular species 1. The mass of 150196 Da is an increase of 1976 Da from the mass of the molecular species 2. The mass of 150356 Da is an increase of 1975 Da from the mass of the molecular species 3. These differences in mass correspond to the mass of the FAM-DBCO-N₃-KGG added by the reaction of the compound D (988.03 Da) or 2 times of the mass, and thus coupling of the first FAM-DBCO-N₃-KGG followed by coupling of the second FAM-DBCO-N₃-KGG was observed.

Also at pH 8.9, the molecular species 1 to 3 were decreased, while molecular species with masses of 149077 Da, 149235 Da, 149399 Da, 150062 Da, 150224 Da, and 150386 Da were newly increased. The mass of 149077 Da is an increase of 1019 Da from the mass of the molecular species 1. The mass of 149235 Da is an increase of 1015 Da from the mass of the molecular species 2. The mass of 149399 Da is an increase of 1018 Da from the mass of the molecular species 3. The mass of 150062 Da is an increase of 2004 Da from the mass of the molecular species 1. The mass of 150224 Da is an increase of 2004 Da from the mass of the molecular species 2. The mass of 150386 Da is an increase of 2005 Da from the mass of the molecular species 3. These differences in mass almost correspond to the mass of the FAM-DBCO-N₃-KGG coupled by the reaction of the compound D (988.03 Da) or 2 times of the mass, and thus coupling of the first FAM-DBCO-N₃-KGG followed by coupling of the second FAM-DBCO-N₃-KGG was observed. From the above, it was shown that the compound D was reacted with the humanized monoclonal antibody at a pH of 8.0 or more.

### Test Example 2: Identification of Modified Site with Peptide Map and Mass Spectrometer

Trastuzumab or trastuzumab reacted with the compound B (50 µg, in 25 µL of water) was mixed with 25 µL of 0.2% Rapigest SF (manufactured by Waters Corporation). After adding 2.6 µL of 100 mM dithiothreitol (DTT, final concentration 5 mM), the solution was incubated at 60°C for 30 minutes. After adding 5.8 µL of 150 mM iodoacetamide (final concentration 15 mM), the solution was incubated at room temperature in the dark for 30 minutes. After adding 12.5 µL of 0.2 µg/µL trypsin and incubating the mixture at 37°C for 3.5 hours, 7.85 µL of 5% TFA (final concentration: 0.5%) was added to stop the reaction. After centrifugation, the supernatant was injected into an ACQUITY UPLC Peptide BEH130 C18 column (1.7 µm, 2.1 × 150 mm, manufactured by Waters Corporation) connected to an ACQUITY UPLC H-Class FTN system (manufactured by Waters Corporation), and peptide mapping was performed. Elution was performed with a 2 to 90% acetonitrile gradient with 0.1% formic acid, and the temperature of the column oven was kept at 60°C. Mass spectrometry of the separated peptide was performed with an Xevo G2-XS QTof spectrometer (manufactured by Waters Corporation) connected to an ACQUITY UPLC H-Class FTN system (manufactured by Waters Corporation), and analysis of the obtained mass spectrum was performed with MassLynx ver. 4.1 or UNIFI 1.9.2 software.

### (Results)

Fig. 15 shows the results of the peptide mapping. The results for trastuzumab (tCAP conjugate) reacted with the compound B and the results for trastuzumab are shown in the upper and lower areas, respectively. As a result of analyzing the mass of each peak, the peak (T20) eluted at 44.65 minutes observed with trastuzumab showed a mass of 2844.45 Da, and this mass matched the mass of the T20 peptide derived from 226-251 residues of the trastuzumab H chain (2844.46 Da).

| Fragment name | Amino acid sequence |
|---|---|
| T20 | THTCPPCPAPELLGGPSVFLFPPKPKDTLMISR (SEQ ID NO: 154) |

Meanwhile, in the case of the tCAP conjugate, the peak eluted at 44.65 minutes disappeared, and the peak (T19-21) eluted at 50.77 minutes showed a mass of 4461.17 Da. As shown below, this peak matched the mass of 4461.20 obtained by adding the mass of one of N-acetyl azide Lys-Gly-Gly to the mass of the T19-T20-T21 peptide derived from residue numbers 222-258 (219-255 by Eu numbering) of the trastuzumab H chain.

| Fragment name | Amino acid sequence |
|---|---|
| T19-21 | SCDKTHTCPPCPAPELLGGPSVFLFPPKPKDTLMISR (SEQ ID NO: 155) |

The above showed that one of N-acetyl(azide Lys)-Gly-Gly was added to the T19-T20-T21 peptide (residue number: 222-258), and a candidate for the addition position was considered to be Lys225, Lys249, or Lys251 (Lys222, Lys246, or Lys248 by Eu numbering). Fig. 16 shows the result of MS/MS for the peak eluted at 50.77 minutes with the tCAP conjugate. By following the mass data of the b-series from the parent mass 4461.178, a mass peak difference (difference between b6 and b3) corresponding to the mass of Lys-Thr-His (residue number: 245-247) was detected, and thus it was indicated that Lys225 was not modified. Meanwhile, by following the mass data of the y-series, a mass peak difference (difference between y11 and y9) corresponding to the mass of Lys-Pro (residue number: 249-248) was detected, and thus it was indicated that the residue number Lys249 (Lys246 by Eu numbering) was not modified. In addition, a mass peak difference (difference between y9 and y7) was detected that corresponded to the mass of Lys-Pro (residue number: 251-250) to which N-acetyl(azide Lys)-Gly-Gly was added. The above strongly indicates that the main modified site by the tCAP reaction is the residue number Lys251 (Lys248 by Eu numbering).

### Example 10: Modification of Azidated Human IgG with Anticancer Drug or Fluorescent Agent

### Reaction of Trastuzumab with Anticancer Drug or Fluorescent Agent

A conjugate was prepared by a click reaction between azidated trastuzumab prepared by reacting the compound B with trastuzumab and an anticancer drug (DM1) or a fluorescent agent (IRDye (trademark) 800CW). First, a reagent for modification of trastuzumab with an anticancer drug was prepared as follows. 50 µL of 100 mM Mertansine (manufactured by MedChemExpress) dissolved in DMSO, 50 µL of 100 mM Bromoacetamido-dPEG (trademark) 4-amido-DBCO (manufactured by Quanta BioDesign, Ltd.) dissolved in DMSO, and 10 µL of a 1 M NaHCO₃ solution (pH 8.9) were mixed, and the mixture was left to stand at room temperature for 1 hour. The DBCO-PEG4-DM1 reagent solution thus prepared was diluted to 1 mM with DMSO, 10 µL of the 1 mM DBCO-PEG4-DM1 reagent solution was mixed with 100 µL of a PBS solution of azidated trastuzumab prepared to 20 µM (at a molar ratio of 5 : 1), and the mixture was left to stand at room temperature for 0.5 hours.

In the modification of trastuzumab with a fluorescent agent, 10 µL of 1 mM IRDye (trademark) 800CW DBCO Infrared Dye (manufactured by LI-COR Biosciences) dissolved in DMSO was mixed with 100 µL of a PBS solution of azidated trastuzumab prepared to 20 µM (at a molar ratio of 5 : 1), and the mixture was left to stand at room temperature for 1 hour. Each reaction solution was sampled, the reaction was stopped with formic acid at a final concentration of 5%, and then the sample was analyzed by LC-MS in the same manner as in Example 3.

### (Results)

Figs. 17 and 18 show the results of LC-MS of the reaction solution after modification of azidated trastuzumab with the anticancer drug and the reaction solution after modification of azidated trastuzumab with the fluorescent agent, respectively. (A), (B), (C), and (D) of Fig. 17 are elution chromatograms (280 nm) of trastuzumab (unmodified antibody), azidated trastuzumab modified with the compound B (azidated KGG-modified antibody), a reaction product of the anticancer drug and azidated trastuzumab, and a reaction product of azidated trastuzumab and the fluorescent agent, respectively.

(A), (B), (C), and (D) of Fig. 18 show results of mass spectrometry of antibodies eluted in the unmodified antibody, the azidated KGG-modified antibody, the reaction product of the anticancer drug and azidated trastuzumab, and the reaction product of azidated trastuzumab and the fluorescent agent, respectively. As shown in Fig. 18(A), in the unmodified antibody, three molecular species with masses of 148058 Da, 148220 Da, and 148381 Da were observed due to the diversity of sugar chains. As a result of the modification with the compound B, as shown in Fig. 18(B), azidated KGG was coupled in a monovalent or bivalent form, and molecular species with masses of 148374 Da, 148531 Da, and 148682 Da were generated in the monovalent form. In the bivalent form, molecular species with masses of 148682 Da (overlapping with the peak in the monovalent form), 148841 Da, and 149003 Da were generated. From the peak ratio in Fig. 18(B), the production ratio after the reaction was calculated between a 0-valent (unmodified) form : monovalent form : bivalent form, and was determined to be 0 : 29 : 71.

When the azidated KGG-modified antibody was reacted with DBCO-PEG4-DM1 at a molar ratio of 5, molecular species to which DM1 was added in a monovalent or bivalent form were generated as shown in Fig. 18(C). Molecular species with masses of 149672 Da, 149833 Da, and 149994 Da are modifications in the monovalent form, and molecular species with masses of 151283 Da, 151447 Da, and 151609 Da are modifications in the bivalent form.

When the azidated KGG-modified antibody was reacted with IRDye (trademark) 800CW DBCO Infrared Dye having a molar ratio of 5, molecular species to which IRDye (trademark) 800CW was added in a monovalent or bivalent form were generated as shown in Fig. 18(D). Molecular species with masses of 149630 Da, 149792 Da, and 149954 Da are modifications in the monovalent form, and molecular species with masses of 151202 Da, 151365 Da, and 151526 Da are modifications in the bivalent form.

As described above, if the azidated KGG-modified antibody is used, the antibody can be highly functionalized easily by adding a functional ligand to which DBCO group is added to the antibody by a click reaction.

### Test Example 3: Evaluation of Affinity of Azidated KGG-Modified Antibody with Fc Receptor

Affinity analysis of the azidated KGG-modified antibody to Fc receptors (FcγRI, FcγRIIa, FcγRIIb, FcγRIIIa, and FcγRIIIb) was performed using BIAcore 8K (manufactured by Cytiva) at 25°C. An anti-His tag antibody (manufactured by Cytiva) was immobilized on a CM5 sensor chip with an amine coupling method to an amount of about 8000 in RU value. To this resulting product, an Fc receptor (FcgRI-His, FcgRIIaI-His, FcgRIIbI-His, FcgRIIIaI-His, or FcgRIIIbI-His, manufactured by Sino Biological, Inc.) was further injected at a concentration of 0.4 ug/mL for immobilization to an amount of about 100 in RU value. As an analyte, the azidated KGG-modified antibody or the unmodified antibody dissolved in a PBST buffer solution (PBS, 0.005% Tween 20) at 8 concentrations in total (in the case of FcγRI, 16 concentrations) from 1600 nM to 12.5 nM (in the case of FcγRI, concentrations from 1600 nM to 0.05 nM ) was injected at a flow rate of 30 µL/min to obtain a sensorgram (association time: 150 seconds, dissociation time: 250 seconds) (in the case of FcγRI, association time: 120 seconds, dissociation time: 480 seconds). The dissociation constant (Kd) was calculated with a 1 : 1 binding model using the attached BIA evaluation software.

Meanwhile, affinity analysis of the azidated KGG-modified antibody to FcRn (fetal Fc receptor) was performed using BIAcore 8K (manufactured by Cytiva) at 25°C as follows. FcRn (manufactured by Sino Biological, Inc.) prepared to a concentration of 1.0 ug/mL using a sodium acetate pH 5.5 solution (manufactured by Cytiva) was injected into a CM5 sensor chip activated for amine coupling reaction, and immobilized to an amount of about 300 in RU value. As an analyte, the azidated KGG-modified antibody or the unmodified antibody dissolved in a phosphate buffer solution (50 mM sodium phosphate, 150 mM NaCl, pH 6.0) at 8 concentrations in total from 1600 nM to 12.5 nM was injected at a flow rate of 30 µL/min to obtain a sensorgram (association time: 120 seconds, dissociation time: 150 seconds, regeneration: 30 seconds). The dissociation constant (Kd) was calculated with a bivalent binding model using the attached BIA evaluation software. In the regeneration solution, 100 mM Tris HCl and 0.2 M NaCl, pH 8.0, were used.

### (Results)

The results of evaluating the affinity of the azidated KGG-modified antibody and the affinity of the unmodified antibody with the Fc receptor are shown in the following table. Except for FcγRIIb, significant difference was not seen between the affinity of the azidated KGG-modified antibody and the affinity of the unmodified antibody to the Fc receptor. This indicates that the modification using the compound B has almost no influence on binding to the Fc receptor important for the effector function of the IgG antibody and thus is useful as a conjugate technology.

| Receptor | Kd to unmodified antibody (nM) | Kd to azidated KGG-modified antibody (nM) |
|---|---|---|
| FcγRI | 5.3 | 6.6 |
| FcγRIIa | 100 | 88 |
| FcγRIIb | 270 | 2000 |
| FcγRIIIa | 66 | 65 |
| FcγRIIIb | 4000 | 3200 |
| FcRn | 570 | 250 |

The above-described embodiments are for describing the present invention, and do not limit the scope of the present invention. That is, the scope of the present invention is indicated by the claims, not by the embodiments. Various modifications made within the scope of the claims and within the scope of the meaning of an invention equivalent to the claims are regarded as being within the scope of the present invention.

This application is based on Japanese Patent Application No. 2021-146504 filed on September 8, 2021. The entire description, claims, and drawings of Japanese Patent Application No. 2021-146504 are incorporated herein by reference.

The present invention is useful for production of a modified antibody, production of a complex containing an antibody, and a research reagent.

## Claims

1. A compound or a salt of the compound, the compound represented by Formula I described below: 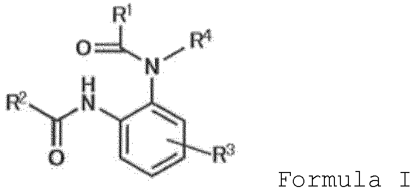 wherein R¹ is a substituent wherein R¹-H has a pKa of 4 to 14,
R² or R³ has an IgG-binding peptide that specifically binds to an Fc region of an IgG,
when R² has the IgG-binding peptide, R³ is absent or selected from the group consisting of substituted or unsubstituted alkyl groups having 1 to 8 carbon atoms, substituted or unsubstituted alkenyl groups having 2 to 8 carbon atoms, substituted or unsubstituted alkynyl groups having 2 to 8 carbon atoms, nitro group, halogens, and carboxamide groups, or
when R³ has the IgG-binding peptide, R² is selected from the group consisting of substituted or unsubstituted alkyl groups having 1 to 8 carbon atoms, substituted or unsubstituted alkenyl groups having 2 to 8 carbon atoms, substituted or unsubstituted alkynyl groups having 2 to 8 carbon atoms, substituted or unsubstituted peptide chains having 2 to 50 amino acid residues, substituted or unsubstituted polymer chains having a polymerization degree of 2 to 50, and combinations thereof, and
R⁴ is H or a substituted or unsubstituted alkyl group having 1 to 8 carbon atoms.

2. The compound or the salt of the compound according to claim 1, wherein
R¹ is nitrophenoxy group.

3. The compound or the salt of the compound according to claim 1 or 2, wherein
R² has the IgG-binding peptide, and
R³ is absent.

4. The compound or the salt of the compound according to claim 1 or 2, wherein
R² is represented by Formula II described below: 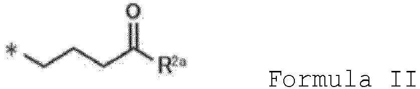 wherein R^{2a} represents the IgG-binding peptide, and
* represents a carbon atom of carbonyl group in Formula I.

5. The compound or the salt of the compound according to claim 3, wherein
the IgG-binding peptide is modified with a drug, a reactive functional group, or a labeling substance.

6. The compound or the salt of the compound according to claim 4, wherein
the IgG-binding peptide is modified with a drug, a reactive functional group, or a labeling substance.

7. The compound or the salt of the compound according to claim 1, wherein
R³ has the IgG-binding peptide, and
R² has a drug, a reactive functional group, or a labeling substance.

8. The compound or the salt of the compound according to claim 2, wherein
R³ has the IgG-binding peptide, and
R² has a drug, a reactive functional group, or a labeling substance.

9. The compound or the salt of the compound according to claim 1 or 2, wherein
R³ is represented by Formula III, IV, or V described below: 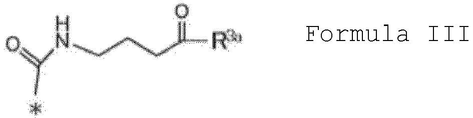 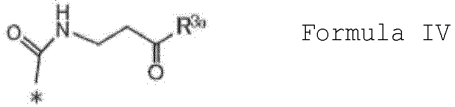 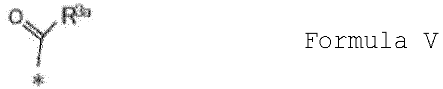 wherein R^{3a} represents the IgG-binding peptide, and
* represents a carbon atom of benzene ring in Formula I.

10. The compound or the salt of the compound according to claim 1 or 2, wherein
the IgG-binding peptide has an amino acid sequence shown in any one of SEQ ID NOs: 1 to 115.

11. The compound or the salt of the compound according to claim 7, wherein
the IgG-binding peptide has an amino acid sequence shown in any one of SEQ ID NOs: 116 to 151.

12. An antibody modification reagent comprising
the compound or the salt of the compound according to claim 1 or 2.

13. A method for producing a modified antibody,
comprising a reaction step of reacting the antibody modification reagent according to claim 12 with an IgG.

14. A modified antibody comprising
a drug, a reactive functional group, or a labeling substance bound to Lys of an Fc region of an IgG in a monovalent or bivalent form via an atomic group containing Lys-Gly-Gly.

15. The modified antibody according to claim 14, wherein
an amino group of the Lys side chain contained in the atomic group is substituted with the drug, the reactive functional group, or the labeling substance.

16. The modified antibody according to claim 14 or 15, wherein
the reactive functional group is azide group.

17. The modified antibody according to claim 14 or 15, wherein
the IgG is a human IgG.

18. The modified antibody according to claim 14 or 15, wherein
the IgG is tocilizumab or trastuzumab.
